# EUROPEAN PATENT SPECIFICATION

(11) **EP 2 361 243 B1**
(45) Date of publication and mention of the grant of the patent: **03.07.2013**
(21) Application number: 09826761.0
(22) Date of filing: 12.11.2009
(51) Int. Cl.: C07C 237/04, C07C 255/46, C07C 317/48, C07C 323/60, C07D 213/32, A61K 31/277, A61P 11/06, A61P 19/02

(54) **HALOALKYL CONTAINING COMPOUNDS AS CYSTEINE PROTEASE INHIBITORS**
HALOGENALKYLHALTIGE VERBINDUNGEN ALS CYSTEINPROTEASEINHIBITOREN
COMPOSÉS CONTENANT UN HALOGÉNOALKYLE EN TANT QU INHIBITEURS DE CYSTÉINE PROTÉASE

(30) Priority: 13.11.2008 US 114369 P
(43) Date of publication of application: 31.08.2011
(73) Proprietor: Virobay, Inc., Menlo Park, CA 94025 (US)
(72) Inventor: HART, Barry, Palo Alto California 94301 (US); GREEN, Michael, Half Moon Bay California 94019 (US); SETTI, Eduardo, San Mateo California 94401 (US); ROEPEL, Michael, Portland Oregon 97206 (US)
(74) Representative: Campbell, Patrick John Henry
(86) International application number: PCT/US2009/064227
(87) International publication number: WO 2010/056877

(56) References cited:
- WO-A1-2006/056047
- WO-A1-2006/060494
- WO-A1-2006/102423
- WO-A1-2006/128287
- WO-A2-2006/102243
- WO-A2-2006/102535
- WO-A2-2008/042968
- CHUN SING LI, ET AL.: "Identification of a potent and selective non-basic cathepsin K inhibitor", BIOORGANIC & MEDICINAL CHEMISTRY LETTERS, vol. 16, no. 7, 18 January 2006 (2006-01-18), pages 1985-1989, XP005330431, Elsevier Science Publishers, Oxford, GB ISSN: 0960-894X, DOI: 10.1016/j.bmcl.2005.12.071

## Description

### BACKGROUND OF THE INVENTION

The present invention is directed to compounds that are inhibitors of cysteine proteases, in particular, cathepsins B, K, L, F, and S and are therefore useful in treating diseases mediated by these proteases. The present invention is directed to pharmaceutical compositions comprising these compounds and processes for preparing them.

### STATE OF THE ART

Cysteine proteases represent a class of peptidases characterized by the presence of a cysteine residue in the catalytic site of the enzyme. Cysteine proteases are associated with the normal degradation and processing of proteins. However, the aberrant activity of cysteine proteases, for example as a result of increased expression or enhanced activation, may have pathological consequences. In this regard, certain cysteine proteases are associated with a number of disease states, including arthritis, muscular dystrophy, inflammation, tumor invasion, glomerulonephritis, malaria, periodontal disease, metachromatic leukodystrophy and others. For example, increased cathepsin B levels and redistribution of the enzyme are found in tumors; thus, suggesting a role for the enzyme in tumor invasion and metastasis. In addition, aberrant cathepsin B activity is implicated in such disease states as rheumatoid arthritis, osteoarthritis, pneumocystis carinii, acute pancreatitis, inflammatory airway disease and bone and joint disorders.

The prominent expression of cathepsin K in osteoclasts and osteoclast-related multinucleated cells and its high collagenolytic activity suggest that the enzyme is involved in osteoclast-mediated bone resorption and, hence, in bone abnormalities such as occurs in osteoporosis. In addition, cathepsin K expression in the lung and its elastinolytic activity suggest that the enzyme plays a role in pulmonary disorders as well.

Cathepsin L is implicated in normal lysosomal proteolysis as well as in several disease states, including, but not limited to, metastasis of melanomas. Cathepsin S is implicated in Alzheimer's disease and certain autoimmune disorders, including, but not limited to juvenile onset diabetes, multiple sclerosis, pemphigus vulgaris, Graves' disease, myasthenia gravis, systemic lupus erythemotasus, rheumatoid arthritis and Hashimoto's thyroiditis. In addition, cathepsin S is implicated in: allergic disorders, including, but not limited to asthma; and allogeneic immune responses, including, but not limited to, rejection of organ transplants or tissue grafts.

In view of the number of diseases wherein it is recognized that an increase in cysteine protease activity contributes to the pathology and/or symptomatology of the disease, molecules which inhibit the activity of this class of enzymes, in particular molecules that inhibit cathepsins B, K, L, F, and/or S, will therefore be useful as therapeutic agents.

WO 2006/060494 describes haloalkyl containing compounds as cysteine protease inhibitors. WO 2006/102243 describes alpha ketoamide compounds as cysteine protease inhibitors. WO 2006/128287 describes fluoroalkylamine derivatives as cathepsin inhibitors. Chun Sing Li et al Bio-organic Medicinal Chemistry Letters 2006, 16(7), 1985-1989 describes identification of a potent and selective non-basic cathepsin K inhibitor.

### SUMMARY OF THE INVENTION

In one aspect, this invention is directed to a compound of Formula I: 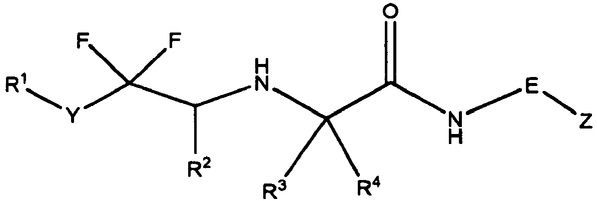 wherein:

R¹ is aryl or heteroaryl, wherein the aryl ring or heteroaryl ring is optionally substituted by one, two, or three groups independently selected from alkyl, halo, hydroxy, hydroxyalkyl, alkoxy, alkoxyalkyl, haloalkyl, haloalkoxy, cyano, nitro, acyl, aryl, aryloxy, arylsulfonyl, heteroaryl, heteroaryloxy, heteroarylsulfonyl, heterocyclyl, heterocyclyloxy, cycloalkyl, cycloalkyloxy, carboxy, allcoxycarbonyl, alkylsulfonyl, alkylsulfinyl, aminosulfonyl, and aminoalkyl;

Y is oxygen or -S(O)m, in which m is 0, 1, or 2;

R² is hydrogen, R⁷-C≡C-, or cis or trans R⁷-CH=CH-, in which R⁷ is lower alkyl of 1-3 carbon atoms or cycloalkyl of 3-6 carbon atoms;

R³ is hydrogen or lower alkyl of 1-3 carbon atoms;

R⁴ is -A-X-R⁸, in which A is alkylene of 1-3 carbon atoms optionally substituted by one, two, or three groups independently selected from alkyl and halo, X is a covalent bond, oxygen or -S(O)ₙ, in which n is 0, 1 or 2, and R⁸ is hydrogen, alkyl, haloalkyl, cycloalkyl, cycloalkylalkyl, aryl, aralkyl, heteroaryl, heteroaralkyl, heterocyclyl, or heterocyclylalkyl, all of which are optionally substituted with 1, 2, or 3 substituents selected from the group consisting of lower alkyl of 1-4 carbon atoms, halo, and cyano;

with the proviso that R⁸ cannot be hydrogen when X is oxygen or -S(O)ₙ;

E is a covalent bond or -CH(R⁹)-C(O)-C(O)-NH-, in which R⁹ is hydrogen or lower alkyl of 1-6 carbon atoms optionally substituted by one, two, three, or four groups independently selected from halo and cycloalkyl of 3-7 carbon atoms;

Z is -C(R⁵)(R⁶)-R¹⁰; in which

R⁵ is hydrogen or lower alkyl of 1-4 carbon atoms; and

R⁶ is hydrogen, lower alkyl of 1-4 carbon atoms, cycloalkyl, or aryl; or

R⁵ and R⁶ when taken together with the carbon to to which they are attached form a cycloalkyl group of 3-6 carbon atoms optionally substituted by lower alkyl of 1-4 carbon atoms, halo, or hydroxyl; and;

R¹⁰ is hydrogen or cyano;

with the proviso that when E is a covalent bond, R¹⁰ cannot be hydrogen; and with the proviso that when E is -CH(R⁹)-C(O)-C(O)-NH-, R¹⁰ cannot be cyano.

or a pharmaceutically salt or N-oxide thereof.

In a second aspect, this invention is directed to a pharmaceutical composition comprising a compound of Formula I or a pharmaceutically acceptable salt or N-oxide thereof and at least one pharmaceutically acceptable excipient.

The present invention also provides a pharmaceutical composition as defined above for use in a method for treating a disease in an animal mediated by Cathepsin B, K, L, F or S, which method comprises administering to the animal said pharmaceutical composition optionally in admixture with one or more suitable excipients. Optionally the disease is asthma, arthritis, atherosclerosis, COPD, MS, or psoriasis.

The present invention also provides a compound of Formula I or a pharmaceutically acceptable salt or N-oxide thereof for use in the treatment of the human or animal body by therapy.

The therapy may be e.g. a method for treating a disease in an animal mediated by cysteine proteases, which method comprises administering to the animal a therapeutically effective amount of a compound of Formula I or a pharmaceutically acceptable salt thereof

Processes are described for preparing compounds of Formula I and pharmaceutically acceptable salts or prodrugs thereof.

A method is described of treating a patient undergoing a therapy wherein the therapy causes an immune response, in particular a deleterious immune response, in the patient, comprising administering to the patient a compound of Formula I or a pharmaceutically acceptable salt thereof. Typically, the immune response is mediated by MHC class II molecules. The compounds of this invention can be administered prior to, simultaneously, or after the therapy. The therapy may involve treatment with a biologic.

This invention is directed to the use of a compound of Formula I or a pharmaceutically acceptable salt thereof for the manufacture of a medicament.

### DETAILED DESCRIPTION OF THE INVENTION

### Definitions:

Unless otherwise stated, the following terms used in the specification and claims are defined for the purposes of this Application and have the following meanings.

"Alkyl" represented by itself means a straight or branched, saturated aliphatic radical containing 1, 2, 3, 4, 5, or 6 carbon atoms, unless otherwise indicated e.g., methyl, ethyl, n-propyl, isopropyl, butyl, *sec*-butyl, isobutyl, *tert*-butyl, n-pentyl, n-hexyl, and the like.

"Alkylene", unless indicated otherwise, means a straight or branched, saturated aliphatic, divalent radical having 1, 2, 3, 4, 5, or 6 carbon atoms, e.g., methylene (-CH₂-), ethylene (-CH₂CH₂-), trimethylene (-CH₂CH₂CH₂-), tetramethylene (-CH₂CH₂CH₂CH₂-) 2-methyltetramethylene (-CH₂CH(CH₃)CH₂CH₂-), pentamethylene (-CH₂CH₂CH₂CH₂CH₂-), and the like.

"Amino" means an -NH₂ radical.

"Alkylamino" or "dialkylamino" refers to a -NHR and NRR' radical respectively, where R and R' are independently alkyl groups as defined above e.g., methylamino, dimethylamino, and the like.

"Alkoxy" refers to an -OR radical where R is an alkyl group as defined above e.g., methoxy, ethoxy, and the like.

"Alkoxycarbonyl" refers to a -C(O)OR radical where R is an alkyl group as defined above e.g., methoxycarbonyl, ethoxycarbonyl, and the like.

"Alkoxycarbonylalkyl" means an -(alkylene)-C(O)OR radical where R is alkyl as defined above e.g., methoxycarbonylmethyl, 2-, or 3-ethoxycarbonylmethyl, and the like.

"Alkoxyalkyl" means a linear monovalent hydrocarbon radical of 1, 2, 3, 4, 5, or 6 carbon atoms or a branched monovalent hydrocarbon radical of 3, 4, 5, or 6 carbons substituted with at least one alkoxy group, preferably one or two alkoxy groups, as defined above, e.g., 2-methoxy-ethyl, 1-, 2-, or 3-methoxypropyl, 2-ethoxyethyl, and the like.

"Alkoxyalkyloxyalkyl" refers to a -(alkylene)-O-(alkylene)-OR radical where R is an alkyl group as defined above, e.g., 2-methoxyethyloxymethyl, 3-methoxypropyloxyethyl, and the like.

"Aminoalkyl" means a linear monovalent hydrocarbon radical of 1, 2, 3, 4, 5, or 6 carbon atoms or a branched monovalent hydrocarbon radical of 3, 4, 5, or 6 carbon atoms substituted with at least one, preferably one or two, -NRR' where R is hydrogen, alkyl, or - COR^{a} where R^{a} is alkyl, and R' is hydrogen or alkyl as defined above e.g., aminomethyl, methylaminoethyl, dimethylaminoethyl, 1,3-diaminopropyl, acetylaminopropyl, and the like.

"Aminosulfonyl" refers to an -SO₂R radical where R is -NRR' where R is hydrogen, alkyl, or -COR^{a} where R^{a} is alkyl, and R' is hydrogen or alkyl as defined above e.g., aminosulfonyl, methylaminosulfonyl, dimethylaminosulfonyl, and the like.

"Alkylthio" refers to an -SR radical where R is an alkyl group as defined above e.g., methylthio, ethylthio, and the like.

"Alkylsulfinyl" refers to an -S(O)R radical where R is an alkyl group as defined above e.g., methylsulfinyl, ethylsulfinyl, and the like.

"Alkylsulfonyl" refers to an -SO₂R radical where R is an alkyl group as defined above e.g., methylsulfonyl, ethylsulfonyl, and the like.

"Acyl" refers to a -COR radical where R is hydrogen, alkyl, haloalkyl, aryl, aralkyl, heteroaryl, heteroaralkyl, or heterocyclyl as defined herein, e.g., formyl, acetyl, trifluoroacetyl, benzoyl, piperazin-1-ylcarbonyl, and the like.

"Aminocarbonyl" refers to a -CONRR' radical where R is hydrogen or alkyl and R' hydrogen, alkyl, aryl, aralkyl, heteroaryl, or heteroaralkyl.

"Animal" includes humans, non-human mammals (e.g., dogs, cats, rabbits, cattle, horses, sheep, goats, swine, deer, and the like) and non-mammals (e.g., birds, and the like).

"Aromatic" refers to a moiety wherein the constituent atoms make up an unsaturated ring system, all atoms in the ring system are *sp*² hybridized and the total number of pi electrons is equal to 4n+2.

"Aryl" refers to a monocyclic or fused bicyclic ring assembly containing 6, 7, 8, 9 or 10 ring carbon atoms wherein each ring is aromatic e.g., phenyl or naphthyl.

"Aralkyl" refers to an -(alkylene)-R radical where R is aryl as defined above e.g., benzyl, phenethyl, and the like.

"Aryloxy" refers to an -OR radical where R is aryl as defined above e.g., phenoxy, and the like.

"Aryloxyalkyl" refers to an -(alkylene)-OR radical where R is aryl as defined above e.g., phenoxymethyl, 2-, or 3-phenoxymethyl, and the like

"Arylsulfonyl" refers to an -SO₂R radical where R is an aryl group as defined above e.g., phenylsulfonyl, and the like.

"Biologic" means a therapeutic agent originally derived from living organisms for the treatment or management of a disease. examples include, but are not limited to, proteins (recombinant and plasma derived), monoclonal or polyclonal, humanized or murine antibodies, toxins, hormones, Remicade^{®}, Refacto^{®}, Referon-A^{®}, Factor VIII, Factor VII, Betaseron^{®}, Epogen^{®}, Enbrel^{®}, Interferon beta, Botox^{®}, Fabrazyme^{®}, Elspar^{®}, Cerezyme^{®}, Myobloc^{®}, Aldurazyme^{®}, Verluma^{®}, Interferon alpha, Humira^{®}, Aranesp^{®}, Zevalin^{®} or OKT3 and the like. Biologics are currently available for the treatment of a variety of diseases such as cancer, rheumatoid arthritis, and haemophilia.

"Carboxy" refers to the -C(O)OH radical.

"Carboxyalkyl" refers to an -(alkylene)-C(O)OH radical e.g., carboxymethyl, carboxyethyl, and the like.

"Cycloalkyl" refers to a monovalent saturated or partially unsaturated, monocyclic ring containing 3, 4, 5, 6, 7, or 8 ring carbon atoms e.g., cyclopropyl, cyclobutyl, cyclopentyl, cyclohexyl, cyclohexenyl, 2,5-cyclohexadienyl, and the like.

"Cycloalkyloxy" refers to a radical -O-R where R is cycloalkyl group as defined above, e.g cyclopropyloxy, cyclohexyloxy, cyclopentyloxy, and the like.

"Cycloalkylalkyl" refers to an -(alkylene)-R radical where R is cycloalkyl as defined above e.g., cyclopropylmethyl, cyclobutylethyl, cyclobutylmethyl, and the like

"Cycloalkylene" refers to a divalent saturated or partially unsaturated monocyclic ring containing 3, 4, 5, 6, 7, or 8 ring carbon atoms. For example, the instance wherein "R¹ and R² together with the carbon atom to which both R¹ and R² are attached form cycloalkylene" includes, but is not limited to, the following: 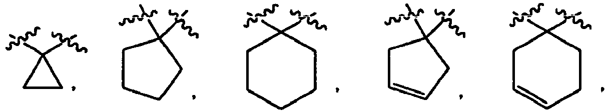 and the like.

"Disubstituted amino" refers to a -NRR' radical where R is alkyl, aryl, aralkyl, heteroaryl, heteroaralkyl, or heterocyclyl and R' is alkyl, aryl, aralkyl, heteroaryl, heteroaralkyl, cycloalkyl, heterocyclyl, cycloalkylalkyl, hydroxyalkyl, alkoxyalkyl, or acyl as defined herein. Representative examples include, but are not limited to, dimethylamino, methylphenylamino, benzylmethylamino, acetylmethylamino, and the like.

"Disease" specifically includes any unhealthy condition of an animal or part thereof and includes an unhealthy condition that may be caused by, or incident to, medical or veterinary therapy applied to that animal, i.e., the "side effects" of such therapy.

"Deleterious immune response" means an immune response that prevents effective treatment of a patient or causes disease in a patient. As an example, dosing a patient with a murine antibody either as a therapy or a diagnostic agent causes the production of human antimouse antibodies that prevent or interfere with subsequent treatments. The incidence of antibody formation versus pure murine monoclonal can exceed 70%. (*see* Khazaeli, M. B. et al. J. Immunother. 1994, 15 , pp 42-52; Dillman R. O. et al. Cancer Biother. 1994, 9, pp 17-28; and Reinsberg, J. Hybridoma. 1995,14, pp 205-208). Additional examples of known agents that suffer from deleterious immune responses are blood-clotting factors such as factor VIII. When administered to hemophilia A patients, factor VIII restores the ability of the blood to clot. Although factor VIII is a human protein, it still elicits an immune response in hemophiliacs as endogenous factor VIII is not present in their blood and thus it appears as a foreign antigen to the immune system. Approximately 29-33% of new patients will produce antibodies that bind and neutralize the therapeutically administered factor VIII (*see* Lusher J. M. Semin Thromb Hemost. 2002, 28(3), pp 273-276). These neutralizing antibodies require the administration of larger amounts of factor VIII in order to maintain normal blood clotting parameters; an expensive regimen of treatment in order to induce immune tolerance (*see* Briet E et al. Adv. Exp. Med. Bio. 2001, 489, pp 89-97). Another immunogenic example is adenoviral vectors. Retroviral therapy remains experimental and is of limited utility. One reason is that the application of a therapeutic virus generates an immune response capable of blocking any subsequent administration of the same or similar virus *(see* Yiping Yang et al. J. of Virology. 1995, 69, pp 2004-2015). This ensures that retroviral therapies must be based on the transient expression of a protein or the direct incorporation of viral sequence into the host genome. Directed research has identified multiple viral neutralizing epitopes recognized by host antibodies *(see* Hanne, Gahery-Segard et al. J. of Virology 1998. 72, pp 2388-2397) suggesting that viral modifications will not be sufficient to overcome this obstacle. This invention will enable a process whereby an adenoviral therapy will have utility for repeated application. Another example of an immunogenic agent that elicits neutralizing antibodies is the well-known cosmetic agent Botox. Botulin toxin protein, is purified from the fermentation of *Clostridium botulinum.* As a therapeutic agent, it is used for muscle disorders such as cervical dystonia in addition to cosmetic application. After repeated exposure patients generate neutralizing antibodies to the toxin that results in reduced efficacy *(see* Birklein F. et al. Ann Neurol. 2002, 52, pp 68-73 and Rollnik, J. D. et al. Neurol. Clin. Neurophysiol. 2001, 2001(3), pp 2-4). A "deleterious immune response" also encompasses diseases caused by therapeutic agents. A specific example of this is the immune response to therapy with recombinant human erythropoietin (EPO). Erythropoeitin is used to stimulate the growth or red cells and restore red blood cell counts in patients who have undergone chemotherapy or dialysis. A small percentage of patients develop antibodies to EPO and subsequently are unresponsive to both therapeutically administered EPO and their own endogenous EPO (*see* Casadevall, N. et al., NEJM. 2002, 346, pp 469-475). They contract a disorder, pure red cell aplasia, in which red blood cell production is severely diminished *(see* Gershon S. K. et. al. NEJM. 2002, 346, pp 1584-1586). This complication of EPO therapy is lethal if untreated. Another specific example is the murine antibody, OKT3 (a.k.a., Orthoclone) a monoclonal antibody directed towards CD-3 domain of activated T-cells. In clinical trials 20-40% of patients administered OKT3 produce antibodies versus the therapy. These antibodies besides neutralizing the therapy also stimulate a strong host immune reaction. The immune reaction is severe enough that patients with high titers of human antimouse antibodies are specifically restricted from taking the drug (*see* Orthoclone package label). A final example is a human antibody therapeutic. Humira^{®} is a monoclonal antibody directed against TNF and is used to treat rheumatoid arthritis patients. When taken alone ∼12% of patients develop neutralizing antibodies. In addition, a small percentage of patients given the drug also contract a systemic lupus erthematosus-like condition that is an IgG-mediated immune response induced by the therapeutic agent (*see* Humira package label). Another example of "deleterious immune response" is a host reaction to small molecule drugs. It is known to those skilled in the art that certain chemical structures will conjugate with host proteins to stimulate immune recognition (see Ju. C. et al. 2002. Current Drug Metabolism 3, pp 367-377 and Kimber I. et al. 2002, Toxicologic Pathology 30, pp 54-58.) A substantial portion of this host reactions are IgG mediated. Specific "deleterious immune responses" that are IgG mediated and include: hemolytic anemia, Steven-Johnson syndrome and drug induced Lupus.

"Halo" refers to fluoro, chloro, bromo or iodo.

"Haloalkyl" refers to alkyl as defined above substituted by one or more, preferably 1, 2, 3, 4, or 5 "halo" atoms, as such terms are defined above. Haloalkyl includes monohaloalkyl, dihaloalkyl, trihaloalkyl, perhaloalkyl and the like e.g. chloromethyl, dichloromethyl, difluoromethyl, trifluoromethyl, 2,2,2-trifluoroethyl, perfluoroethyl, 2,2,2-trifluoro-1,1-dichloroethyl, and the like.

"Haloalkylene" refers to alkylene radical as defined above where one to six hydrogen atoms are replaced by chlorine or fluorine atoms(s), preferably one or two hydrogens are replaced with fluoroine or chlorine atoms, more preferably two atoms on the same carbon of the alkylene chain are replaced with fluorine atoms e.g. dichloromethylene, difluoromethylene, 1,2-difluoroethylene, and the like.

"Haloalkoxy" refers to a -OR radical where R is a haloalkyl group as defined above e.g., trifluoromethoxy, 2,2,2-trifluoroethoxy, difluoromethoxy, and the like.

"Heteroaryl" as a group or part of a group denotes an aromatic monocyclic or multicyclic moiety of 5, 6, 7, 8, 9, or 10 ring atoms in which one or more, preferably one, two, or three, of the ring atom(s) is(are) selected from nitrogen, oxygen or sulfur, the remaining ring atoms being carbon. Representative heteroaryl rings include, but are not limited to, pyrrolyl, furanyl, thienyl, oxazolyl, isoxazolyl, thiazolyl, imidazolyl, triazolyl, tetrazolyl, pyridinyl, pyrimidinyl, pyrazinyl, pyridazinyl, indolyl, benzofuranyl, benzothienyl, benzimidazolyl, quinolinyl, isoquinolinyl, quinazolinyl, quinoxalinyl, pyrazolyl, and the like.

"Heteroaralkyl" refers to a -(alkylene)-R radical where R is heteroaryl as defined above e.g., pyridinylmethyl, 1- or 2-furanylethyl, imidazolylmethyl, and the like.

"Heteroaryloxyalkyl" refers to an-(alkylene)-OR radical where R is heteroaryl as defined above e.g., furanyloxymethyl, 2-, or 3-indolyloxyethyl, and the like.

"Heteroaryloxy" refers to an -OR radical where R is heteroaryl as defined above.

"Heteroaralkyloxy" refers to an -OR radical where R is heteroaralkyl as defined above.

"Heteroarylsulfonyl" refers to an -SO₂R radical where R is an heteroaryl group as defined above e.g., pyridinylsulfonyl, and the like.

"Heterocyclyl" refers to a saturated or partially unsaturated, mono or bicyclic radical of 4, 5 or 6 carbon ring atoms wherein one or more, preferably one, two, or three of the ring carbon atoms are replaced by a heteroatom selected from -N=, -N-, -O-, -S-, -SO-, or -S(O)₂-and further wherein one or two ring atoms are optionally replaced by a keto (-CO-) group. The heterocyclyl ring is optionally fused to aryl or heteroaryl ring as defined herein. Representative examples include, but are not limited to, imidazolidinyl, morpholinyl, thiomorpholinyl, thiomorpholino-1-oxide, thiomorpholino-1,1-dioxide, tetrahydropyranyl, tetrahydrothiopyranyl, 1-oxo-tetrahydrothiopyranyl, 1,1-dioxotetrathiopyranyl, indolinyl, piperazinyl, piperidyl, pyrrolidinyl, pyrrolinyl, quinuclidinyl, and the like.

"Heterocyclylalkyl" refers to a -(alkylene)-heterocyclyl radical as defined in this Application. Representative examples include, but are not limited to, imidazolidin-1-ylmethyl, morpholin-4-ylmethyl, thiomorpholin-4-ylmethyl, thiomorpholin-4-ylmethyl-1-oxide, indolinylethyl, piperazinylmethyl or ethyl, piperidylmethyl or ethyl, pyrrolidinylmethyl or ethyl, and the like.

"Heterocyclyloxy" refers to an -OR radical where R is heterocyclyl as defined above e.g., piperidinyloxy, tetrahydrofuranyloxy, and the like.

"Heterocyclylsulfonyl" refers to an -SO₂R radical where R is an heterocyclyl group as defined above e.g., piperidinylsulfonyl, piperazinylsulfonyl, and the like.

"Heterocyclylalkylene" refers to a divalent heterocyclyl group, as defined in this Application, e.g., the instance wherein R¹ and R² together with the carbon atom to which both R¹ and R² are attached form heterocyclylalkylene" includes, but is not limited to, the following: 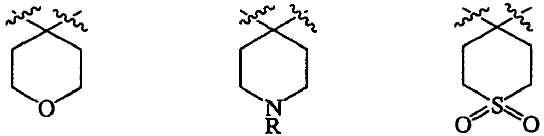 in which R is a substituent defined in the Summary of the Invention

"Hydroxy" means -OH radical.

"Hydroxyalkyl" means a linear monovalent hydrocarbon radical of 1, 2, 3, 4, 5, or 6 carbon atoms or a branched monovalent hydrocarbon radical of 3, 4, 5, 6 carbons substituted with one or two hydroxy groups, provided that if two hydroxy groups are present they are not both on the same carbon atom. Representative examples include, but are not limited to, hydroxymethyl, 2-hydroxyethyl, 2-hydroxypropyl, 3-hydroxypropyl, 1-(hydroxymethyl)-2-methylpropyl, 2-hydroxybutyl, 3-hydroxybutyl, 4-hydroxybutyl, 2,3-dihydroxypropyl, 1-(hydroxymethyl)-2-hydroxyethyl, 2,3-dihydroxybutyl, 3,4-dihydroxybutyl and 2-(hydroxymethyl)-3-hydroxypropyl, preferably 2-hydroxyethyl, 2,3-dihydroxypropyl, and 1-(hydroxymethyl)-2-hydroxyethyl.

"Isomers" mean compounds having identical molecular formulae but differ in the nature or sequence of bonding of their atoms or in the arrangement of their atoms in space. Isomers that differ in the arrangement of their atoms in space are termed "stereoisomers". Stereoisomers that are not mirror images of one another are termed "diastereomers" and stereoisomers that are nonsuperimposable mirror images are termed "enantiomers" or sometimes "optical isomers". A carbon atom bonded to four nonidentical substituents is termed a "chiral center". A compound with one chiral center has two enantiomeric forms of opposite chirality is termed a "racemic mixture". A compound that has more than one chiral center has 2^{*n*-1} enantiomeric pairs, where *n* is the number of chiral centers. Compounds with more than one chiral center may exist as either an individual diastereomer or as a mixture of diastereomers, termed a "diastereomeric mixture". When one chiral center is present a stereoisomer may be characterized by the absolute configuration of that chiral center. Absolute configuration refers to the arrangement in space of the substituents attached to the chiral center. Enantiomers are characterized by the absolute configuration of their chiral centers and described by the *R*- and *S*-sequencing rules of Cahn, Ingold and Prelog. Conventions for stereochemical nomenclature, methods for the determination of stereochemistry and the separation of stereoisomers are well known in the art (e.g., see "Advanced Organic Chemistry", 4th edition, March, Jerry, John Wiley & Sons, New York, 1992). It is understood that the names and illustration used in this Application to describe compounds of Formula (I) are meant to be encompassed all possible stereoisomers.

"Keto" means a -C(O) radical.

"Monosubstituted amino" refers to an -NHR radical where R is alkyl, aryl, aralkyl, heteroaryl, heteroaralkyl, cycloalkyl, cycloalkylalkyl, hydroxyalkyl, alkoxyalkyl, or acyl as defined herein. Representative examples include, but are not limited to, methylamino, phenylamino, benzylamino, cycloalkylmethylamino, acetylamino, trifluoroacetyl, and the like.

"Nitro" means -NO₂ radical.

"Optional" or "optionally" or "may be" means that the subsequently described event or circumstance may or may not occur, and that the description includes instances where the event or circumstance occurs and instances in which it does not. For example, the phrase "wherein the aromatic ring in R^{a} is optionally substituted with one or two substituents independently selected from alkyl" means that the aromatic ring may or may not be substituted with alkyl in order to fall within the scope of the invention.

The present invention also includes *N*-oxide derivatives of a compound of Formula I. *N*-oxide derivative mean a compound of Formula I in which a nitrogen atom is in an oxidized state (i.e., N→O) e.g., pyridine *N*-oxide, and which possess the desired pharmacological activity.

"Pathology" of a disease means the essential nature, causes and development of the disease as well as the structural and functional changes that result from the disease processes.

"Pharmaceutically acceptable" means that which is useful in preparing a pharmaceutical composition that is generally safe, non-toxic and neither biologically nor otherwise undesirable and includes that which is acceptable for veterinary use as well as human pharmaceutical use.

"Pharmaceutically acceptable salts" means salts of compounds of Formula I which are pharmaceutically acceptable, as defined above, and which possess the desired pharmacological activity. Such salts include acid addition salts formed with inorganic acids such as hydrochloric acid, hydrobromic acid, sulfuric acid, nitric acid, phosphoric acid, and the like; or with organic acids such as acetic acid, propionic acid, hexanoic acid, heptanoic acid, cyclopentanepropionic acid, glycolic acid, pyruvic acid, lactic acid, malonic acid, succinic acid, malic acid, maleic acid, fumaric acid, tartaric acid, citric acid, benzoic acid, *o*-(4-hydroxybenzoyl)benzoic acid, cinnamic acid, mandelic acid, methylsulfonic acid, ethanesulfonic acid, 1,2-ethanedisulfonic acid, 2-hydroxy-ethanesulfonic acid, benzenesulfonic acid, p-chlorobenzenesulfonic acid, 2-naphthalenesulfonic acid, *p*-toluenesulfonic acid, camphorsulfonic acid, 4-methylbicyclo[2.2.2]oct-2-ene-1-carboxylic acid, glucoheptonic acid, 4,4'-methylenebis(3-hydroxy-2-ene-1-carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid and the like.

Pharmaceutically acceptable salts also include base addition salts which may be formed when acidic protons present are capable of reacting with inorganic or organic bases. Acceptable inorganic bases include sodium hydroxide, sodium carbonate, potassium hydroxide, aluminum hydroxide and calcium hydroxide. Acceptable organic bases include ethanolamine, diethanolamine, triethanolamine, tromethamine, *N*-methylglucamine and the like.

Prodrugs of a compound of Formula I are described. Prodrug means a compound that is convertible *in vivo* by metabolic means (e.g. by hydrolysis) to a compound of Formula I. For example an ester of a compound of Formula I containing a hydroxy group may be convertible by hydrolysis in vivo to the parent molecule. Alternatively an ester of a compound of Formula I ontaining a carboxy group may be convertible by hydrolysis in vivo to the parent molecule. Suitable esters of compounds of Formula I containing a hydroxy group, are for example acetates, citrates, lactates, tartrates, malonates, oxalates, salicylates, propionates, succinates, fumarates, maleates, methylene-bis-b-hydroxynaphthoates, gentisates, isethionates, di-*p*-toluoyltartrates, methylsulphonates, ethanesulphonates, benzenesulphonates, p-toluenesulphonates, cyclohexylsulphamates and quinates. Suitable esters of compounds of Formula I containing a carboxy group, are for example those described by Leinweber, F.J. Drug Metab. Res., 1987, 18, page 379. An especially useful class of esters of compounds of Formula I containing a hydroxy group, may be formed from acid moieties selected from those described by Bundgaard et al., J. Med. Chem., 1989, 32, pp 2503-2507, and include substituted (aminomethyl)-benzoates, for example, dialkylamino-methylbenzoates in which the two alkyl groups may be joined together and/or interrupted by an oxygen atom or by an optionally substituted nitrogen atom, e.g. an alkylated nitrogen atom, more especially (morpholino-methyl)benzoates, e.g. 3- or 4-(morpholinomethyl)-benzoates, and (4-alkylpiperazin-1-yl)benzoates, e.g. 3- or 4-(4-alkylpiperazin-1-yl)benzoates.

"Protected derivatives" means derivatives of compounds of Formula I in which a reactive site or sites are blocked with protecting groups. Protected derivatives of compounds of Formula I are useful in the preparation of compounds of Formula I or in themselves may be active cathepsin S inhibitors. A comprehensive list of suitable protecting groups can be found in T.W. Greene, Protective Groups in Organic Synthesis, 3rd edition, John Wiley & Sons, Inc. 1999.

"Therapeutically effective amount" means that amount which, when administered to an animal for treating a disease, is sufficient to effect such treatment for the disease.

"Treatment" or "treating" means any administration of a compound of the present invention and includes:

(1) preventing the disease from occurring in an animal which may be predisposed to the disease but does not yet experience or display the pathology or symptomatology of the disease,

(2) inhibiting the disease in an animal that is experiencing or displaying the pathology or symptomatology of the diseased (i.e., arresting further development of the pathology and/or symptomatology), or

(3) ameliorating the disease in an animal that is experiencing or displaying the pathology or symptomatology of the diseased (i.e., reversing the pathology and/or symptomatology).

"Treatment" or "treating" with respect to combination therapy i.e., use with a biologic means any administration of a compound of the present invention and includes:

(1) preventing the immune response from occurring in an animal which may be predisposed to the immune response but does not yet experience or display the pathology or symptomatology of the immune response,

(2) inhibiting the immune response in an animal that is experiencing or displaying the pathology or symptomatology of the immune response (i.e., arresting further development of the pathology and/or symptomatology), or

(3) ameliorating the immune response in an animal that is experiencing or displaying the pathology or symptomatology of the immune response (i.e., reducing in degree or severity, or extent or duration, the overt manifestations of the immune response or reversing the pathology and/or symptomatology e.g., reduced binding and presentation of antigenic peptides by MHC class II molecules, reduced activation of T-cells and B-cells, reduced humoral and cell-mediated responses and, as appropriate to the particular immune response, reduced inflammation, congestion, pain, necrosis, reduced loss in the efficacy of a biologic agent, and the like).

### PREFERRED EMBODIMENTS

Certain compounds of Formula I within the broadest scope set forth in the Summary of the Invention are preferred. For example:

One preferred group of compounds of Formula I includes those compounds in which R¹ is optionally substituted aryl, R⁴ is A-X-R⁸, and Y is oxygen. Within this group, a subgroup includes compounds in which R² and R³ are both hydrogen and E is a covalent bond, particularly where Z is -C(R⁵)(R⁶)-R¹⁰, in which R⁵ and R⁶ together with the carbon atom to which they are attached form a cycloalkyl group of 3-6 carbon atoms, especially where the cycloalkyl group is cyclopropyl, and R¹⁰ is cyano. Preferred among these compounds are those in which R¹ is phenyl substituted by fluoro, A is alkylene of 1-3 carbon atoms optionally substituted by fluoro, X is a covalent bond or -S(O)₂, and R⁸ is heteroarylalkyl or cycloalkyl.

A second preferred group includes those compounds of Formula I in which E is -CH(R⁹)-C(O)-C(O)-NH- and Z is -C(R⁵)(R⁶)-R¹⁰, in which R⁵ and R⁶ together with the carbon tom to which they are attached form a cycloalkyl group of 3-6 carbon atoms, particularly where the cycloalkyl group is cyclopropyl, and R¹⁰ is hydrogen. Preferred among these compounds are those in which R¹ is phenyl substituted by fluoro, A is alkylene of 1-3 carbon atoms optionally substituted by fluoro, X is a covalent bond or -S(O)₂, R⁸ is heteroaryl or cycloalkyl, and R⁹ is ethyl.

A third preferred group includes those compounds in which R² is R⁷-C ≡C-, R³ is hydrogen, and E is a covalent bond. Within this group preferred compounds include those compounds in which Z is -C(R⁵)(R⁶)-R¹⁰, in which R⁵ and R⁶ together with the carbon atom to which they are attached form a cycloalkyl group of 3-6 carbon atoms, especially where the cycloalkyl group is cyclopropyl, and R¹⁰ is hydrogen. Within this subgroup are preferred compounds in which R¹ is phenyl substituted by fluoro, thiomethyl, or methylsulfonyl, A is alkylene of 1-3 carbon atoms optionally substituted by fluoro, X is a covalent bond or -S(O)₂, and R⁸ is heteroarylalkyl or cycloalkyl, especially where R⁷ is methyl.

Preferred compounds include the following:

(R)-N-(1-cyanocyclopropyl)-2-(2,2-difluoro-2-(4-fluorophenoxy)ethylamino)-3-(pyridin-3-ylmethylsulfonyl)propanamide;

(S)-N-cyclopropyl-3-((R)-2-(2,2-difluoro-2-(4-fluorophenoxy)ethylamino)-3-(pyridin-3-ylmethylsulfonyl)propanamido)-2-oxopentanamide;

(S)-N-cyclopropyl-3-((R)-3-(cyclopropylmethylsulfonyl)-2-(2,2-difluoro-2-(4-fluorophenoxy)ethylamino)propanamido)-2-oxopentanamide;

(R)-N-(1-cyanocyclopropyl)-3-(cyclopropylmethylthio)-2-((S)-1,1-difluoro-1-(4-(methylthio)phenoxy)pent-3-yn-2-ylamino)propanamide;

(R)-N-(1-cyanocyclopropyl)-3-(cyclopropylmethylsulfonyl)-2-((S)-1,1-difluoro-1-(4-(methylsulfonyl)phenoxy)pent-3-yn-2-ylamino)propanamide;

(R)-N-(1-cyanocyclopropyl)-2-((S)-1,1-difluoro-1-(4-(methylthio)phenoxy)pent-3-yn-2-ylamino)-3-(pyridin-2-ylmethylthio)propanamide;

(R)-N-(1-cyanocyclopropyl)-2-((S)-1,1-difluoro-1-(4-(methylsulfonyl)phenoxy)pent-3-yn-2-ylamino)-3-(pyridin-2-ylmethylsulfonyl)propanamide;

(R)-N-(1-cyanocyclopropyl)-3-((S)-1,1-difluoro-1-(4-(methylthio)phenoxy)pent-3-yn-2-ylamino)-2-((pyridin-3-ylmethylthio)methyl)propanamide;

(R)-N-(1-cyanocyclopropyl)-3-((S)-1,1-difluoro-1-(4-(methylsulfonyl)phenoxy)pent-3-yn-2-ylamino)-2-((pyridin-3-ylmethylsulfonyl)methyl)propanamide;

3-(((R)-3-(1-cyanocyclopropylamino)-2-(((S)-1,1-difluoro-1-(4-(methylsulfonyl)phenoxy)pent-3-yn-2-ylamino)methyl)-3-oxopropylsulfonyl)methyl)pyridine 1-oxide;

(S)-N-(1-cyanocyclopropyl)-5-cyclopropyl-2-(2,2-difluoro-2-(4-fluorophenoxy)ethylamino)-4,4-difluoropentanamide; and

(S)-5-cyclopropyl-N-((S)-1-(cyclopropylamino)-1,2-dioxopentan-3-yl)-2-(2,2-difluoro-2-(4-fluorophenoxy)ethylamino)-4,4-difluoropentanamide.

### GENERAL SYNTHETIC SCHEME

Compounds of this invention can be made by the methods depicted in the reaction schemes shown below.

The starting materials and reagents used in preparing these compounds are either available from commercial suppliers such as Aldrich Chemical Co., (Milwaukee, Wis.), Bachem (Torrance, Calif.), or Sigma (St. Louis, Mo.) or are prepared by methods known to those skilled in the art following procedures set forth in references such as Fieser and Fieser's Reagents for Organic Synthesis, Volumes 1-17 (John Wiley and Sons, 1991); Rodd's Chemistry of Carbon Compounds, Volumes 1-5 and Supplementals (Elsevier Science Publishers, 1989); Organic Reactions, Volumes 1-40 (John Wiley and Sons, 1991), March's Advanced Organic Chemistry, (John Wiley and Sons, 4th Edition) and Larock's Comprehensive Organic Transformations (VCH Publishers Inc., 1989).

These schemes are merely illustrative of some methods by which the compounds of this invention can be synthesized, and various modifications to these schemes can be made and will be suggested to one skilled in the art having referred to this disclosure.

The starting materials and the intermediates of the reaction may be isolated and purified if desired using conventional techniques, including but not limited to filtration, distillation, crystallization, chromatography and the like. Such materials may be characterized using conventional means, including physical constants and spectral data.

Unless specified to the contrary, the reactions described herein take place at atmospheric pressure over a temperature range from about -78 °C to about 150 °C, more preferably from about 0 °C to about 125 °C and most preferably at about room (or ambient) temperature, e.g., about 20 °C. In the reactions described hereinafter it may be necessary to protect reactive functional groups, for example hydroxy, amino, imino, thio or carboxy groups, where these are desired in the final product, to avoid their unwanted participation in the reactions. Conventional protecting groups may be used in accordance with standard practice, for examples see T.W. Greene and P. G. M. Wuts in "Protective Groups in Organic Chemistry" John Wiley and Sons, 1999.

The preparation of compounds of Formula I is shown below.

### 1) Preparation of a Compound of Formula I in which R² is Hydrogen, R⁴ is -A-X-R⁸, E is a Covalent Bond, and Z is -C(R⁵)(R⁶)-R¹⁰

Reaction Scheme I shows a synthesis of a compound of Formula I in which R² is hydrogen, R⁴ is -A-X-R⁸, in which X is S or O and R⁸ is as defined above, E is a covalent bond, and Z is

-C(R⁵)(R⁶)-R¹⁰, in which R¹⁰ is cyano.

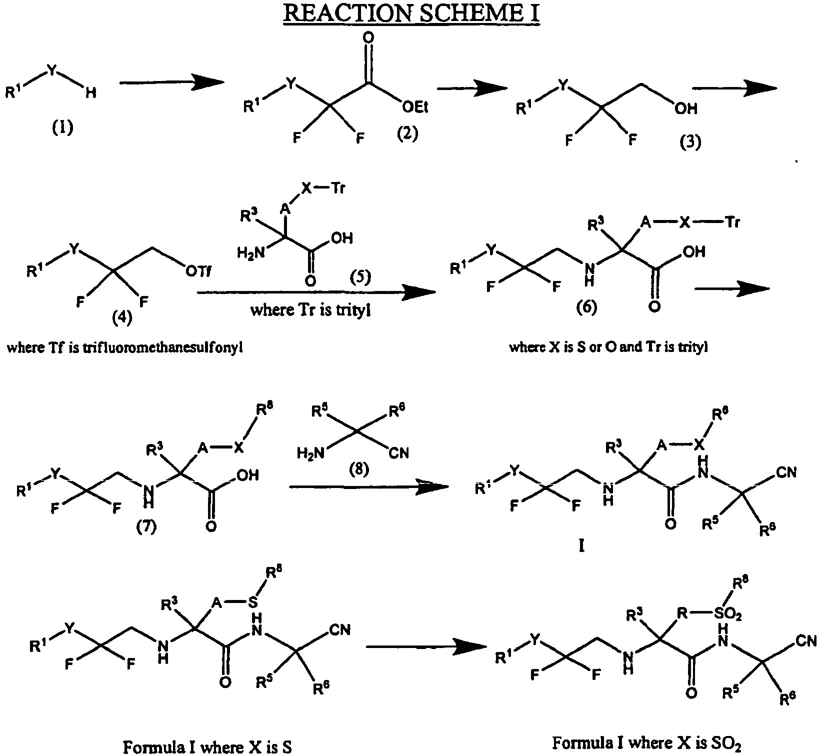

### Step 1 - Preparation of a Compound of Formula (2)

A mixture of a compound of formula (1) in a polar solvent, typically N,N-dimethylformamide, is added to a metal hydride, typically sodium hydride, dispersed in the same solvent, at a temperature of about room temperature. To this mixture is added an ester of 2-bromo-2,2-difluoroacetic acid, preferably ethyl 2-bromo-2,2-difluoroacetate, optionally in the same solvent. The mixture is stirred for about 2-3 hours at room temperature. When the reaction is substantially complete, the product of formula (2) is isolated by conventional means, for example by addition of ethyl acetate, careful addition of water to quench the reaction, washing with water, and removing the solvent under reduced pressure to provide a compound of formula (2).

### Step 2 - Preparation of a Compound of Formula (3)

To a mixture of a compound of formula (2) in an inert solvent, typically diethyl ether, is added a reducing agent, preferably lithium aluminum hydride, at a temperature of about 0°C. The mixture is then stirred at about room temperature for about 4 hours. When the reaction is substantially complete, the product of formula (3) is isolated by conventional means, for example by addition of ethyl acetate to quench the excess lithium aluminum hydride, followed by methanol and excess hydrochloric acid. Extraction with an inert solvent, typically ethyl acetate, and removing the solvent under reduced pressure provides a compound of formula (3).

### Steps 3 and 4 - Preparation of a Compound of Formula (6)

To a mixture of a compound of formula (3) and a hindered base, typically diisopropylethylamine, in an inert solvent, typically dichloromethane, at a temperature of about -78°C, is added trifluoromethanesulfonic anhydride. The mixture is then stirred for about 1-3 hours to form a compound of formula (4). To this product is added a mixture of a compound of formula (5) in an inert solvent, typically dichloromethane, and the temperature allowed to warm to 0°C. When the reaction is substantially complete, the product of formula (6) is isolated by conventional means, for example by addition of ethyl acetate, washing with water, and removing the solvent under reduced pressure provides a compound of formula (6).

### Step 5 - Preparation of a Compound of Formula (7)

To a mixture of a compound of formula (6) in an inert solvent, typically dichloromethane, is added trifluoroacetic acid, followed by triethylsilane, at about room temperature. The mixture is then stirred at about room temperature for about 4 hours, and solvent removed under reduced pressure. To the residue is added hexanes and aqueous sodium hydroxide solution, and the mixture extracted with hexanes. To the sodium hydroxide layer is added a compound of formula R⁸-halo, preferably R⁸Br, and optionally a phosphine derivative, for example P(CH₂CH₂CO₂H)₃. When the reaction is substantially complete, the product of formula (7) is isolated by conventional means, for example by addition of ethyl acetate, and washing with a dilute acid, for example hydrochloric acid, and removing the solvent under reduced pressure.

### Step 6 - Preparation of a Compound of Formula I

The compound of formula (7) is contacted with a compound of formula (8) in the presence of reagents suitable for amide formation, for example 1-methyl-3-(3'-dimethylaminopropyl)-carbodiimide (EDCI) and 1-hydroxybenzotriazole (HOBT), in the presence of a mild base, for example N-methylmorpholine NMM, in an inert organic solvent, for example dichloromethane. The reaction is conducted at about room temperature, for about 2-12 hours. When the reaction is substantially complete, the product of Formula I is isolated by conventional means, for example by diluting the reaction mixture with an inert organic solvent, for example ethyl acetate, and washing with aqueous sodium bicarbonate, brine and drying, to provide a compound of Formula I in which R² is hydrogen, R⁴ is -A-X-R⁸, in which X is S or O and R⁸ is as defined above, and E is a covalent bond.

### Step 7 - Preparation of a Compound of Formula I where X is SO₂

To prepare a compound of Formula I where X is -SO₂-, the product from Step 6 dissolved in a suitable solvent, for example N-methylpyrrolidine, is contacted with Oxone in water. Typically the reaction is conducted at about room temperature.. When the reaction is substantially complete, the product of Formula I where X is -SO₂- is isolated by conventional means, for example by diluting the reaction mixture with an inert organic solvent, for example ethyl acetate, and washing with aqueous sodium bicarbonate, brine and drying, to provide a compound of Formula I in which R² is hydrogen, R⁴ is -A-X-R⁸, in which X is SO₂ and R⁸ is as defined above, and E is a covalent bond.

### 2) Preparation of a Compound of Formula I in which R² is Hydrogen, R⁴ is -A-X-R⁸, E is -CH(R⁹)-C(O)-C(O)-NH-, and Z is -C(R⁵)(R⁶)-R¹⁰

The compounds of Formula I in which R² is hydrogen, R⁴ is A-X-R⁸, E is -CH(R⁹)-C(O)-C(O)-NH-, and Z is -C(R⁵)(R⁶)R¹⁰, in which R¹⁰ is hydrogen, are prepared as shown in Reaction Scheme II.

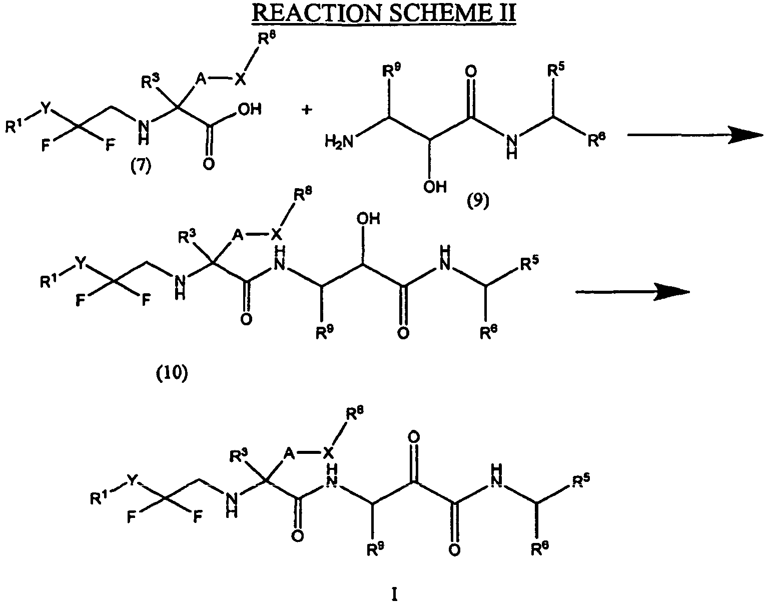

### Step 1 - Preparation of a Compound of Formula (10)

The compound of formula (7), prepared as shown in Reaction Scheme I, is contacted with a compound of formula (9) in the presence of reagents suitable for amide formation, for example 1-ethyl-3-(3'-dimethylaminopropyl)-carbodiimide (EDCI) and 1-hydroxybenzotriazole (HOBT), in the presence of a mild base, for example N-methylmorpholine NMM, in an inert organic solvent, for example dichloromethane. The reaction is conducted at about room temperature, for about 2-12 hours. When the reaction is substantially complete, the product of Formula I is isolated by conventional means, for example by diluting the reaction mixture with an inert organic solvent, for example ethyl acetate, and washing with aqueous sodium bicarbonate, brine and drying, to provide a compound of formula (10).

### Step 2 - Preparation of a Compound of Formula I

The compound of formula (10) is is reacted with a mild oxidizing agent, for example 1,1,1-triacetoxy-1,1-dihydro-1,2-benziodoxol-3(1H)-one (Dess Martin periodinanane Reagent). The reaction is conducted in an inert organic solvent, for example dichloromethane, for about 1 hour at about room temperature. When the reaction is substantially complete, the product of Formula I is isolated by conventional means, for example by diluting the reaction mixture with an inert organic solvent, for example ethyl acetate, and washing with dilute sodium bicarbonate, water, brine and drying. Removal of the solvent under reduced pressure provides a compound of Formula I in which R² is hydrogen, R⁴ is -X-R⁸, E is -CH(R⁹)-C(O)-C(O)-NH-, and Z is -C(R⁵)(R⁶)R¹⁰, in which R¹⁰ is hydrogen

### 2A) Preparation of a Compound of Formula I in which R² is Hydrogen, R⁴ is -A-SO₂-R⁸, E is -CH(R⁹)-C(O)-C(O)-NH-, and Z is -C(R⁵)(R⁶)-R¹⁰, in which R¹⁰ is Hydrogen

The compounds of Formula I in which R² is hydrogen, R⁴ is -X-R⁸, in which X is - SO₂, E is -CH(R⁹)-C(O)-C(O)-, and Z is -C(R⁵)(R⁶)-R¹⁰, in which R¹⁰ is hydrogen, are prepared as shown in Reaction Scheme IIA.

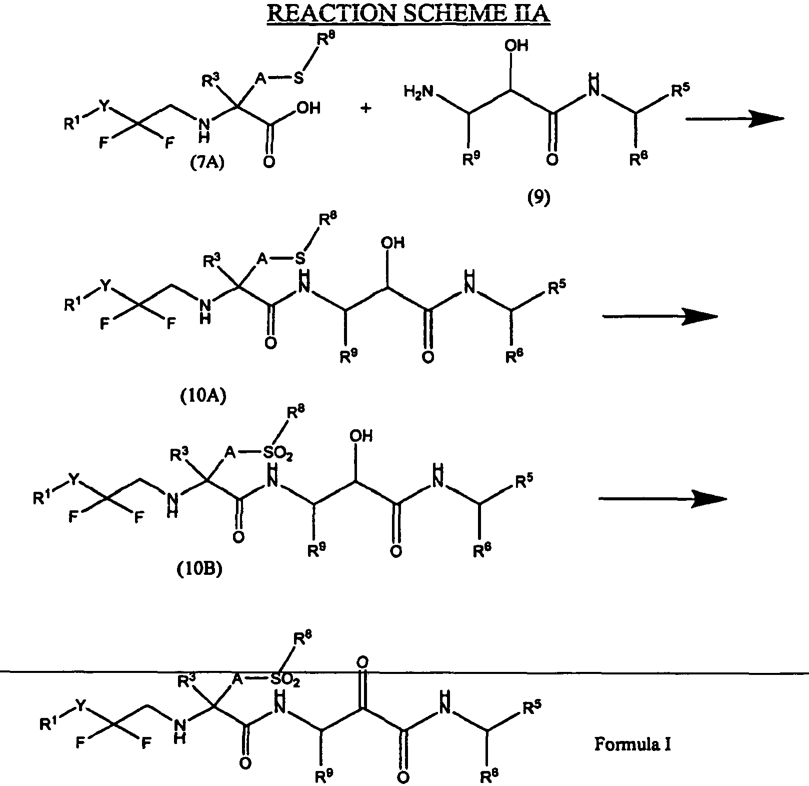

### Step 1 Preparation of a Compound of Formula (10A)

The compound of (7A), which is a compound of formula (7) in which X is -S-, is reacted as shown in step 1 in Reaction Scheme II with a compound of formula (9), to give a compound of formula (10A).

### Step 2 Preparation of a Compound of Formula (10B)

The compound of formula (10A) is dissolved in an amine, typically N-methylmorpholine, and an aqueous solution of Oxone (potassium peroxymonosulfate) added, and the mixture stirred at room temperature for about 3 hours. When the reaction is substantially complete, the product of formula (10B) is isolated by conventional means, and used in the next reaction with no further purification.

### Step 3 Preparation of a Compound of Formula I in which X is -SO₂-

The compound of formula (10B) is then reacted as shown in Reaction Scheme II, step 2, to provide a compound of Formula I in which X is -SO₂-, E is -CH(R⁹)-C(O)-C(O)-NH-, and Z is -C(R⁵)(R⁶)-R¹⁰, in which R¹⁰ is hydrogen.

### 3) Preparation of a Compound of Formula I in which R² is R⁷-C≡C-, R⁴ is -A-X-R⁸, E is a Covalent Bond, and Z is -C(R⁵)(R⁶)-R¹⁰, in which R¹⁰ is Cyano

The compounds of Formula I in which R² is R⁷-C ≡C-, R⁴ is -A-X-R⁸, E is a covalent bond, and Z is -C(R⁵)(R⁶)-R¹⁰, in which R¹⁰ is cyano, are prepared as shown in Reaction Scheme III.

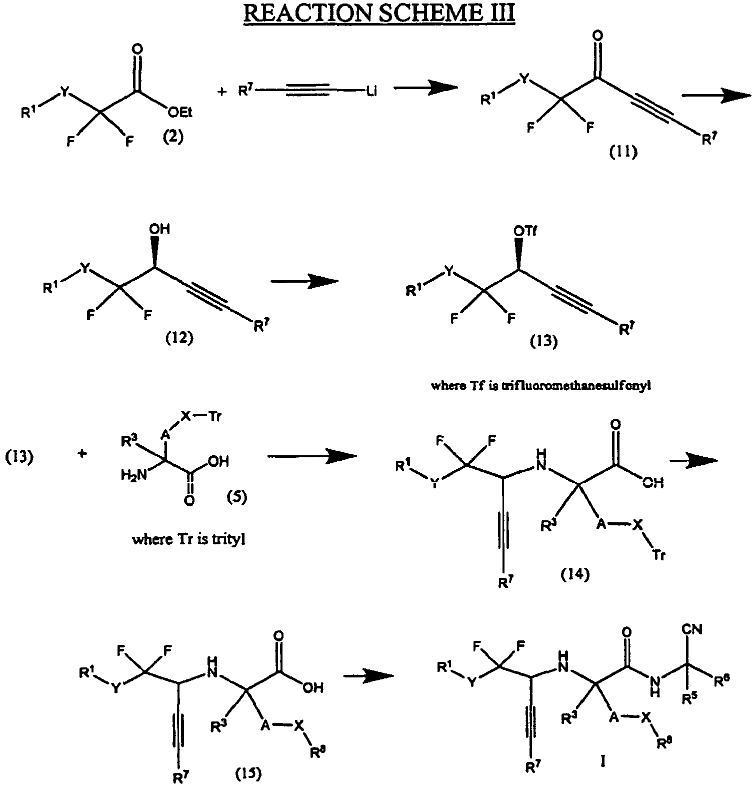

### Step 1 - Preparation of a Compound of Formula (11)

The lithiated alkynyl compound of formula R⁷-C ≡C-Li is prepared by contacting an alkynyl compound of formula R⁷-C ≡C-H with butyl lithium in an inert solvent, typically tetrahydrofuran, at a temperature of about -78°C, for about 10 minutes to 2 hours. The compound of formula (2), prepared for example as shown in Reaction Scheme I, is then added at about -78°C, and the mixture maintained for that temperature for about 20 minutes, then at -40°C for about 5 hours. When the reaction is substantially complete, the product of formula (11) is isolated by conventional means, for example by quenching the reaction, for example by addition of brine or saturated ammonium chloride, then diluting the reaction mixture with an inert organic solvent, for example ether, and washing with brine and drying, to provide a compound of formula (11).

### Step 2 - Preparation of a Compound of Formula (12)

To a solution of a compound of formula (11) in an inert solvent, for example toluene or dichloromethane, or typically a mixture of toluene and dichloromethane, is added the Corey catalyst ((S)-1-methyl-3,3-diphenyl-tetrahydro-pyrrolo[1,2-C][1,3,2]oxazaborazole), at about room temperature. The mixture is cooled to about -60 to about -80°C, and catecholborane added. The reaction is maintained at this temperature for about 10-24 hours, followed by addition of an aqueous strong acid in an inert solvent, typically hydrochloric acid in dioxane, followed by aqueous 10% sodium thiosulfate solution. The mixture is allowed to warm to room temperature. When the reaction is substantially complete, the product of formula (12) is isolated by conventional means, for example by quenching the reaction, for example by addition of brine or saturated ammonium chloride, then diluting the reaction mixture with an inert organic solvent, for example ether, and washing with brine and drying, to provide a compound of formula (12).

### Step 3 - Preparation of a Compound of Formula (14)

To a mixture of the compound of formula (12) in an inert solvent, typically ether, at a temperature of about -10 to 10°C, is added sodium hydride. After about 1 hour trifluoromethanesulfonyl chloride is added, and the mixture stirred for about 1-5 hours to form a compound of formula (13). This solution is cooled to about -78°C, and then a mixture of a compound of formula (5) and a hindered base, typically diisopropylethylamine, in an inert solvent, typically dichloromethane, is added, and the temperature allowed to warm to about 0°C. When the reaction is substantially complete, the product of formula (14) is isolated by conventional means, typically followed by purification on a silica gel column.

### Step 4 - Preparation of a Compound of Formula (15)

To a mixture of a compound of formula (14) in an inert solvent, typically dichloromethane, is added trifluoroacetic acid, followed by triethylsilane, at about room temperature. The mixture is then stirred at about room temperature for about 4 hours, and solvent removed under reduced pressure. To the residue is added hexanes and aqueous sodium hydroxide solution, and the mixture extracted with hexanes. To the sodium hydroxide layer is added a compound of formula R⁸-halo, preferably R⁸Br. When the reaction is substantially complete, the product of formula (15) is isolated by conventional means, for example by addition of ethyl acetate, and washing with a dilute acid, for example hydrochloric acid, and removing the solvent under reduced pressure.

### Step 5 - Preparation of a Compound of Formula I

The compound of formula (15) is contacted with a compound of formula (8), for example aminocyclopropanecarbonitrile, in the presence of reagents suitable for amide formation, for example 1-ethyl-3-(3'-1-dimethylaminopropyl)-carbodiimide (EDCI) and 1-hydroxybenzotriazole (HOBT), in the presence of a mild base, for example N-methylmorpholine NMM, in an inert organic solvent, for example dichloromethane. The reaction is conducted at about room temperature, for about 2-12 hours. When the reaction is substantially complete, the product of Formula I is isolated by conventional means, for example by diluting the reaction mixture with an inert organic solvent, for example ethyl acetate, and washing with aqueous sodium bicarbonate, brine and drying, to provide a compound of Formula I in which in which R² is R⁷-C ≡C-, R⁴ is -A-X-R⁸, E is a covalent bond, and Z is -C(R⁵)(R⁶)-R¹⁰, in which R¹⁰ is cyano.

The compounds of Formula I in which R² is cis or trans R⁷-CH=CH- may be prepared from intermediates of formula (12a) and (12b), the preparation of which is shown in Reaction Scheme IIIA.

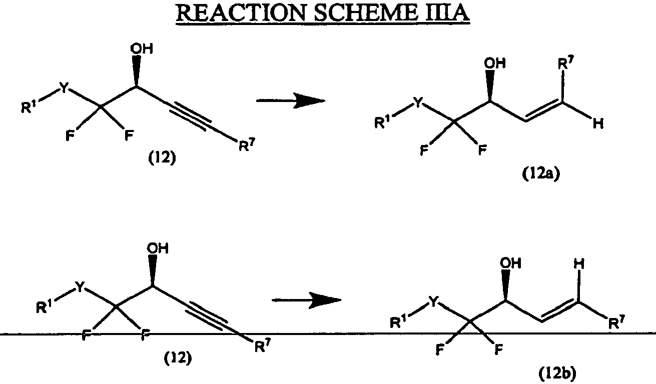

### Preparation of the Compound of Formula (12a)

In general, an alkyne of formula (12) may be reduced to a trans alkene of formula (12a) by means well known in the art. For example, by hydrogenation of the compound of formula (12) using a rhodium complex, for example [RhH₂(OC(O)OH)PPrⁱ₃)₂]. Typically, the reaction is carried out in an inert solvent, for example N,N-dimethylformamide, at about room temperature. When the reaction is substantially complete, the product of formula (12a) is isolated by conventional means.

### Preparation of the Compound of Formula (12b)

In general, an alkyne of formula (12) may be reduced to a cis alkene of formula (12b) by means well known in the art. For example, by hydrogenation of the compound of formula (12) in the presence of a cobalt catalyst, for example [CoH(CO)(PBuⁿ₃)₃. Typically, the reaction is carried out in an inert solvent, for example N,N-dimethylformamide, at about room temperature. When the reaction is substantially complete, the product of formula (12b) is isolated by conventional means.

### 4) Preparation of a Compound of Formula I in which R² is Hydrogen, R⁴ is -A-X-R⁸, in which A is -CH₂-CF₂-, X is a Covalent Bond, and R⁸ is Cycloalkylmethyl, E is a Covalent Bond, and Z is -C(R⁵)(R⁶)-R¹⁰, in which R¹⁰ is Cyano

The compounds of Formula I in which R² is Hydrogen, R⁴ is -A-X-R⁸, in which A is - CH₂-CF₂-, X is a Covalent Bond, and R⁸ is Cycloalkylmethyl, E is a Covalent Bond, and Z is -C(R⁵)(R⁶-R¹⁰, in which R¹⁰ is cyano, are prepared as shown in Reaction Scheme IV.

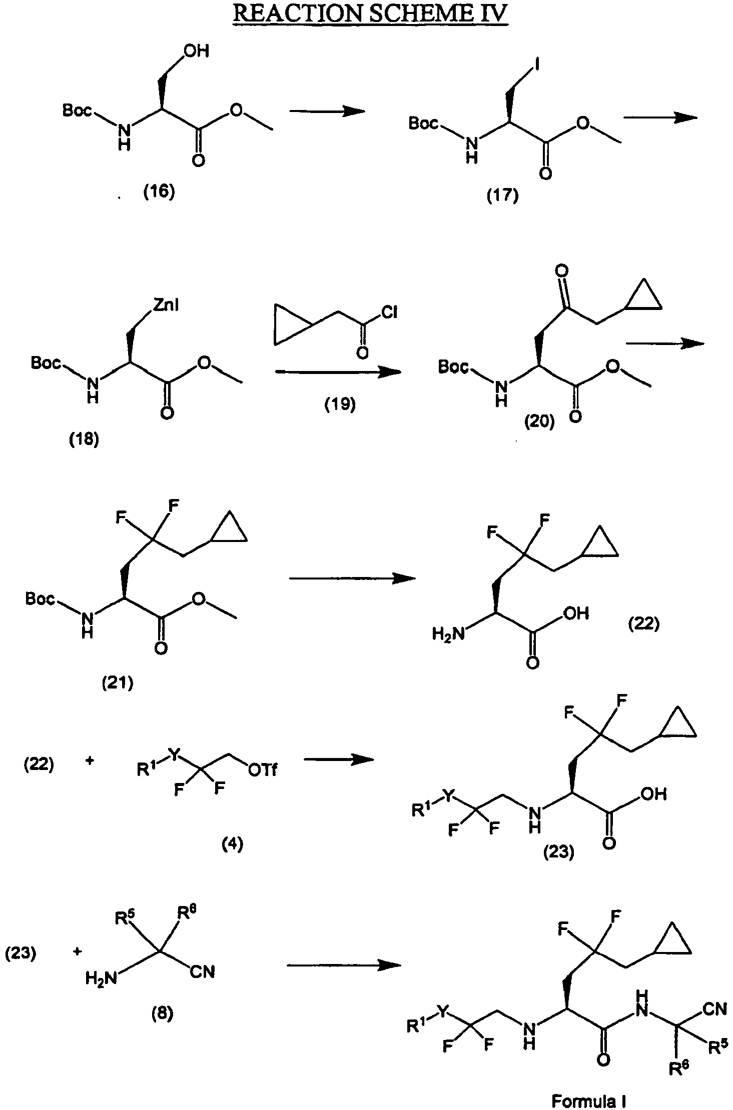

### Step 1-Preparation of a Compound of Formula (17)

Typically, triphenylphosphite methiodide is added to a solution of (*S*)-2-*tert-*butyloxycarbonylamino-3-hydroxypropionic acid methyl ester in a polar solvent, for example N,N-dimethylformamide, at a temperature of about 0°C. When the reaction is substantially complete, typically after about 15-60 minutes, the product of formula (17) is isolated by conventional means.

### Step 2 - Preparation of a Compound of Formula (20)

Zinc dust is suspended in an aqueous acid solution, typically 2M HCl and stirred vigorously for about 5 minutes, then filtered and washed, typically sequentially with water, ethanol, and ether. This material is heated under vacuum for several minutes then allowed to cool to room temperature. The heated/cooling procedure (under vacuum) is typically repeated twice more, and finally the placed under an inert gas, for example nitrogen.

The activated zinc is suspended in a mixture of inert solvents, typically dry benzene and dry N,N-dimethylacetamide, and treated with 1,2-dibromoethane. This mixture is gently heated over 1 hour. Trimethylsilyl chlorideis then added, and the mixture maintained at about room temperature for about 5-60 minutes. When the reaction is substantially complete, the material thus obtained is used in the next reaction without any further purification.

To the mixture of activated zinc obtained is added a solution of (*R*)-2-*tert-*butyloxycarbonylamino-3-iodopropionic acid methyl ester (the compound of formula (17) in a mixture of inert solvents, for example benzene and N,N-dimethylacetamide. The reaction is carried out for about 1-4 hours, and optionally treated with further portions of trimethylsilyl chloride. Palladium bis trityl dichloride (PdCl₂(PPh₃)₂) is then added in a single portion, followed by a solution of cyclopropylacetyl chloride (the compound of formula (19)) in an inert solvent, for example benzene. The reaction was carried out for about 30-60 minutes. When the reaction is substantially complete the product of formula (20) is isolated by conventional means.

### Step 4 - Preparation of a Compound of Formula (21)

To a compound of formula (20), preferably in the absence of solvent, is added diethylaminosulfur trifluoride (DAST), and the resulting mixture maintained at about room temperature for about 5-6 days. When the reaction is substantially complete the product of formula (21) is isolated by conventional means, and optionally purified by column chromatography.

### Step 5 - Preparation of a Compound of Formula (22)

To a compound of formula (21) in an aqueous solvent, typically tetrahydrofuran/water, is added an inorganic base, preferably lithium hydroxide, and the mixture maintained at about room temperature for about 12-24 hours. When the reaction is substantially complete the free acid is isolated by conventional means, for example acidication with an organic acid, typically citric acid, and partitioning between a solvent and water, for example ethyl acetate/water, and removing the solvent under reduced pressure. The BOC group is then removed by treatment with an acid, typically aqueous hydrochloric acid in an inert solvent, for example dioxane, to provide the compound of formula (22) as a hydrochloride salt.

### Step 6 - Preparation of a Compound of Formula (23)

A solution of the difluoroethanol analog of the compound of formula (4) and a hindered base, typically diisopropylethylamine, in an inert solvent, for example dichloromethane, is added to a solution of triflic anhydride in an inert solvent, for example dichloromethane, maintained at about -50 to -80°C. After about 15 minutes, the mixture is allowed to warm to about room temperature, and a compound of formula (22) as its acid salt is added along with a hindered base, typically diisopropylethylamine. The mixture is warmed to about 30-50°C for about 24-72 hours. When the reaction is substantially complete the product of formula (23) is isolated by conventional means, for example acidication with an organic acid, typically citric acid, and partitioning between a solvent and water, for example ethyl acetate/water, and removing the solvent under reduced pressure.

### Step 7 - Preparation of a Compound of Formula I

To a mixture of a compound of formula (23) and an aminonitrile of formula (8) in an inert solvent, for example dichloromethane, is added O-(7-azabenzotriazole-1-yl)-N,N,N,N'-tetramethyluronium hexafluorophosphate (HATU), and the mixture maintained at about room temperature. To this mixture is added a tertiary base, typically N-methylmorpholine, and the mixture maintained at about room temperature for about 12-24 hours. When the reaction is substantially complete the product of Formula I is isolated by conventional means.

### 5) Preparation of a Compound of Formula I in which R² is Hydrogen, R⁴ is -A-X-R⁸, in which A is -CH₂-CF₂-, X is a Covalent Bond, and R⁸ is Cycloalkylmethyl, E is -CH(R⁹)-C(O)-C(O)-NH-, and Z is -C(R⁵)(R⁶)-R¹⁰, where R¹⁰ is Hydrogen

The preparation of a compound of Formula I in which R² is Hydrogen, R⁴ is -A-X-R⁸, in which A is -CH₂-CF₂-, X is a Covalent Bond, and R⁸ is Cycloalkylmethyl, E is -CH(R⁹)-C(O)-C(O)-NH-, and Z is -C(R⁵)(R⁶)-R¹⁰, in which R¹⁰ is hydrogen, from a compound of formula (23) is shown in Reaction Scheme V.

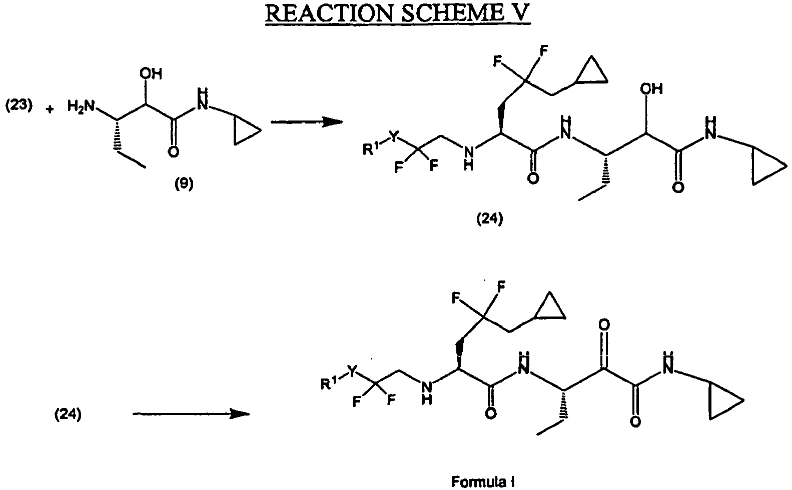

The reaction steps are carried out in a manner similar to those shown in Reaction Scheme II above.

The N-oxides of compounds of Formula I can be prepared by methods known to those of ordinary skill in the art. For example, N-oxides can be prepared by treating an unoxidized form of the compound of Formula I with an oxidizing agent (e.g., trifluoroperacetic acid, permaleic acid, perbenzoic acid, peracetic acid, meta-chloroperoxybenzoic acid, or the like) in a suitable inert organic solvent (e.g., a halogenated hydrocarbon such as dichloromethane) at approximately 0°C. Alternatively, the N-oxides of the compounds of Formula I can be prepared from the N-oxide of an appropriate starting material.

Prodrug derivatives of the compounds of Formula I can be prepared by methods known to those of ordinary skill in the art (e.g., for further details see Saulnier et al. (1994), Bioorganic and Medicinal Chemistry Letters, Vol. 4, p. 1985). For example, appropriate prodrugs can be prepared by reacting a non-derivatized compound of Formula I with a suitable carbamylating agent (e.g., 1,1-acyloxyalkylcarbonochloridate, para-nitrophenyl carbonate, or the like).

Protected derivatives of the compounds of Formula I can be made by means known to those of ordinary skill in the art. A detailed description of the techniques applicable to the creation of protecting groups and their removal can be found in T.W. Greene, Protecting Groups in Organic Synthesis, 3rd edition, John Wiley & Sons, Inc. 1999.

Compounds of the present invention may be conveniently prepared or formed during the process of the invention, as solvates (e.g. hydrates). Hydrates of compounds of the present invention may be conveniently prepared by recrystallisation from an aqueous/organic solvent mixture, using organic solvents such as dioxin, tetrahydrofuran or methanol.

Compounds of Formula I can be prepared as their individual stereoisomers by reacting a racemic mixture of the compound with an optically active resolving agent to form a pair of diastereoisomeric compounds, separating the diastereomers and recovering the optically pure enantiomer. While resolution of enantiomers can be carried out using covalent diasteromeric derivatives of compounds of Formula I, dissociable complexes are preferred (e.g., crystalline diastereoisomeric salts). Diastereomers have distinct physical properties (e.g., melting points, boiling points, solubilities, reactivity, etc.) and can be readily separated by taking advantage of these dissimilarities. The diastereomers can be separated by chromatography or, preferably, by separation/resolution techniques based upon differences in solubility. The optically pure enantiomer is then recovered, along with the resolving agent, by any practical means that would not result in racemization. A more detailed description of the techniques applicable to the resolution of stereoisomers of compounds from their racemic mixture can be found in Jean Jacques Andre Collet, Samuel H. Wilen, Enantiomers, Racemates and Resolutions, John Wiley & Sons, Inc. (1981).

### Pharmacology and Utility

The compounds of the invention are inhibitors of cysteine proteases, in particular, cathepsin S, K, B, and/or F, and accordingly are useful for treating diseases in which cysteine protease activity contributes to the pathology and/or symptomatology of the disease. For example, the compounds of the invention are useful in treating autoimmune disorders, including, but not limited to, juvenile onset diabetes, psoriasis, multiple sclerosis, pemphigus vulgaris, Graves' disease, myasthenia gravis, systemic lupus erythemotasus, rheumatoid arthritis and Hashimoto's thyroiditis, allergic disorders, including, but not limited to, asthma, allogeneic immune responses, including, but not limited to, organ transplants or tissue grafts and endometriosis.

Cathepsin S is also implicated in disorders involving excessive elastolysis, such as chronic obstructive pulmonary disease (e.g., emphysema), bronchiolitis, excessive airway elastolysis in asthma and bronchitis, pneumonities and cardiovascular disease such as plaque rupture and atheroma. Cathepsin S is implicated in fibril formation and, therefore, inhibitors of cathepsins S are of use in treatment of systemic amyloidosis.

The cysteine protease inhibitory activities of the compounds of Formula I can be determined by methods known to those of ordinary skill in the art. Suitable *in vitro* assays for measuring protease activity and the inhibition thereof by test compounds are known. Typically, the assay measures protease-induced hydrolysis of a peptide-based substrate. Details of assays for measuring protease inhibitory activity are set forth in Biological Examples 1-5, *infra.*

### Administration and Pharmaceutical Compositions

In general, compounds of Formula I will be administered in therapeutically effective amounts via any of the usual and acceptable modes known in the art, either singly or in combination with one or more therapeutic agents. A therapeutically effective amount may vary widely depending on the severity of the disease, the age and relative health of the subject, the potency of the compound used and other factors. For example, therapeutically effective amounts of a compound of Formula I may range from about 10 micrograms per kilogram body weight (µg/kg) per day to about 20 milligram per kilogram body weight (mg/kg) per day, typically from about 100 µg/kg/day to about 10 mg/kg/day. Therefore, a therapeutically effective amount for an 80 kg human patient may range from about 1 mg/day to about 1.6 g/day, typically from about 1 mg/day to about 100 mg/day. In general, one of ordinary skill in the art, acting in reliance upon personal knowledge and the disclosure of this Application, will be able to ascertain a therapeutically effective amount of a compound of Formula I for treating a given disease.

The compounds of Formula I can be administered as pharmaceutical compositions by one of the following routes: oral, systemic (e.g., transdermal, intranasal or by suppository) or parenteral (e.g., intramuscular, intravenous or subcutaneous). Compositions can take the form of tablets, pills, capsules, semisolids, powders, sustained release formulations, solutions, suspensions, elixirs, aerosols, or any other appropriate composition and are comprised of, in general, a compound of Formula I in combination with at least one pharmaceutically acceptable excipient. Acceptable excipients are non-toxic, aid administration, and do not adversely affect the therapeutic benefit of the active ingredient. Such excipient may be any solid, liquid, semisolid or, in the case of an aerosol composition, gaseous excipient that is generally available to one of skill in the art. Solid pharmaceutical excipients include starch, cellulose, talc, glucose, lactose, sucrose, gelatin, malt, rice, flour, chalk, silica gel, magnesium stearate, sodium stearate, glycerol monostearate, sodium chloride, dried skim milk, and the like. Liquid and semisolid excipients may be selected from water, ethanol, glycerol, propylene glycol and various oils, including those of petroleum, animal, vegetable or synthetic origin (e.g., peanut oil, soybean oil, mineral oil, sesame oil, and the like). Preferred liquid carriers, particularly for injectable solutions, include water, saline, aqueous dextrose and glycols.

The amount of a compound of Formula I in the composition may vary widely depending upon the type of formulation, size of a unit dosage, kind of excipients and other factors known to those of skill in the art of pharmaceutical sciences. In general, a composition of a compound of Formula I for treating a given disease will comprise from 0.01%w to 10%w, preferably 0.3%w to 1%w, of active ingredient with the remainder being the excipient or excipients. Preferably the pharmaceutical composition is administered in a single unit dosage form for continuous treatment or in a single unit dosage form ad libitum when relief of symptoms is specifically required. Representative pharmaceutical formulations containing a compound of Formula I are described in Example 1 below.

### Examples

The present invention is further exemplified, but not limited by, the following examples that illustrate the preparation of compounds of Formula I (Examples) and intermediates (References) according to the invention.

### Example 1

### Preparation of a Compound of Formula (2)

### A. Preparation of a Compound of Formula (2) in which R¹ is 4-Fluorophenyl and Y is Oxygen

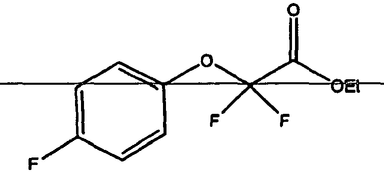

To a mixture of sodium hydride (60%, 535 mg) in N,N-dimethylformamide (30 ml) at room temperature was added a solution of 4-fluorophenol (1.5 g) in a small volume of N,N-dimethylformamide. After 40 minutes ethyl 2-bromo-2,2-difluoroacetate was added dropwise to the mixture, and the mixture stirred for 3 hours. The reaction mixture was then diluted with ethyl acetate, washed 3x with aqueous sodium bicarbonate solution, dried over sodium sulfate, and solvent removed under reduced pressure, to provide crude ethyl 2,2-difluoro-2-(4-fluorophenoxy)acetate, a compound of formula (2). This product was chromatographed on silica gel, eluting with ethyl acetate/hexanes 1/40, to provide pure ethyl 2,2-difluoro-2-(4-fluorophenoxy)acetate, which was used with no further purification.
¹H NMR (CDCl3; 400MHz): δppm 7.05-7.22 (m, 4H); 4.39 (q, 2H), 1.38 (t, 3H)

B. Similarly, following the procedure of Example 1A, but replacing 4-fluorophenol with 4-methylthiophenol, ethyl 2,2-difluoro-2-(4-(methylthio)phenoxy)acetate was prepared.

C. Similarly, following the procedure of Example 1A, but replacing 4-fluorophenol with other compounds of formula (1), other compounds of formula (2) are prepared. For example, ethyl 2,2-difluoro-2-(4-(methylsulfonyl)phenoxy)acetate.

### Example 2

### Preparation of a Compound of Formula (3)

### A. Preparation of a Compound of Formula (3) in which R¹ is 4-Fluorophenyl and Y is Oxygen

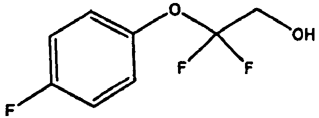

To a solution of ethyl 2,2-difluoro-2-(4-fluorophenoxy)acetate (1.86 g) in diethyl ether (10 ml) at 0°C was added lithium aluminum hydride (1.0M in diethyl ether, 11.9 ml). After 5 minutes the cooling bath was removed and the mixture stirred at room temperature for 4 hours. The mixture was then recooled to 0°C, and ethyl acetate added dropwise, followed by dropwise addition of methanol. The mixture was acidified with aquesous 1N hydrochloric acid, extracted with ethyl acetate, dried over sodium sulfate, and solvent removed under reduced pressure, to provide 2,2-difluoro-2-(4-fluorophenoxy)ethanol as a colorless oil. This product was chromatographed on silica gel, eluting with ethyl acetate/hexanes 1/5, which yielded crystalline 2,2-difluoro-2-(4-fluorophenoxy)ethanol. ¹H NMR (CDC₁₃; 400MHz): δppm 6.91- 7.12 (m, 4H); 3.90 (t, 2H).

B. Similarly, following the procedure of Example 2A, but replacing ethyl 2,2-difluoro-2-(4-fluorophenoxy)acetate with ethyl 2,2-difluoro-2-(4-(methylthio)phenoxy)acetate, 2,2-difluoro-2-(4-(methylthio)phenoxy)ethanol was prepared.

C. Similarly, following the procedure of Example 2A, but replacing ethyl 2,2-difluoro-2-(4-fluorophenoxy)acetate with other compounds of formula (2), other compounds of formula (3) are prepared, for example 2,2-difluoro-2-(4-(methylsulfonyl)phenoxy)ethanol.

### Example 3

### Preparation of a Compound of Formula (4)

### A. Preparation of a Compound of Formula (4), in which R¹ is 4-Fluorophenyl and Y is Oxygen

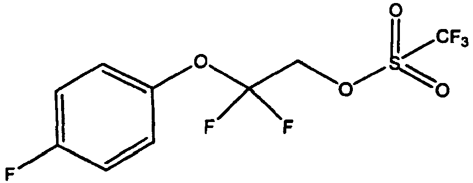

To a solution of 2,2-difluoro-2-(4-fluorophenoxy)ethanol (1.18 g) and diisopropylethylamine (3.74 ml) in dichloromethane at -78°C was added trifluoromethanesulfonic anhydride (1.08 ml) dropwise. After stirring for 4 hours, the solution of 2,2-difluoro-2-(4-fluorophenoxy)ethyl trifluoromethanesulfonate thus produced was used in the next reaction without any purification.

B. Similarly, following the procedure of Example 3A, but replacing 2,2-difluoro-2-(4-fluorophenoxy)ethanol with 2,2-difluoro-2-(4-(methylthio)phenoxy)ethanol, 2,2-diftuoro-2-(4-(methylthio)phenoxy)ethyl trifluoromethanesulfonate was prepared.

C. Similarly, following the procedure of Example 3A, but replacing 2,2-difluoro-2-(4-fluorophenoxy)ethanol with other compounds of formula (3), other compounds of formula (4) are prepared. For example 1-(1,1-difluoro-2-(trifluoromethylsulfonyl)ethoxy)-4-(methylsulfonyl)benzene.

### Example 4

### Preparation of a Compound of Formula (6)

### A. Preparation of a Compound of Formula (6) in which R¹ is 4-Fluorophenyl, Y is Oxygen, R³ is Hydrogen, A is Methylene, and X is Sulfur

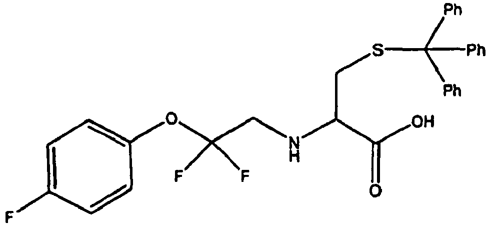

To the product of Example 3 (2,2-difluoro-2-(4-fluorophenoxy)ethyl trifluoromethanesulfonate in dichloromethane) at -78°C was added S-trityl-L-cysteine (2.23 g), and the mixture stirred for 1 hour. The temperature was raised to 0°C, and the mixture stirred for 48 hours. The solvent was removed under reduced pressure, the residue diluted with ethyl acetate, washed with dilute hydrochloric acid (1N), brine, and dried over sodium sulfate. The solvent was removed under reduced pressure, and the residue chromatogrqphed on silica gel, eluting with ethyl acetate/hexane 1/2 containing a few drops of acetic acid, to provide 2-(2,2-difluoro-2-(4-fluorophenoxy)ethylamino)-3-(tritylthio)propanoic acid (2.08 g).

B. Similarly, following the procedure of Example 4A, but replacing 2,2-difluoro-2-(4-fluorophenoxy)ethyl trifluoromethanesulfonate with 2,2-difluoro-2-(4-(methylthio)phenoxy)ethyl trifluoromethanesulfonate, 2-(2,2-difluoro-2-(4-(methylthio)phenoxy)ethylamino)-3-(tritylthio)propanoic acid was prepared.

C. Similarly, following the procedure of Example 4A, but replacing 2,2-difluoro-2-(4-fluorophenoxy)ethyl trifluoromethanesulfonate with with other compounds of formula (4), other compounds of formula (6) are prepared. For example, 2-(2,2-difluoro-2-(4-(methylsulfonyl)phenoxy)ethylamino)-3-(tritylthio)propanoic acid.

### Example 5

### Preparation of a Compound of Formula (7)

### A. Preparation of a Compound of Formula (7) in which R¹ is 4-Fluorophenyl, Y is Oxygen, R³ is Hydrogen, A is Methylene, X is Sulfur, and R⁸ is Cycloproplmethyl

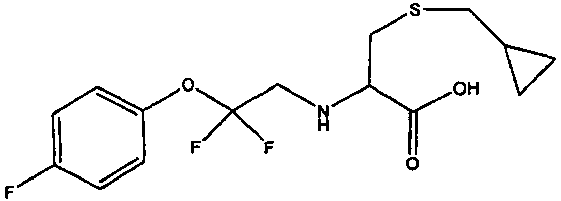

To a solution of 2-(2,2-difluoro-2-(4-fluorophenoxy)ethylamino)-3-(tritylthio)propanoic acid (2.08 g) in dichloromethane (25 ml) was added trifluoroacetic acid (1.2 ml), followed by triethylsilane (1.27 ml). The mixture was strirred for 5 hours, then solvent removed under reduced pressure. Hexanes (100 ml) were added, followed by 2N sodium hydroxide (25 ml), and the mixture stirred. The aqueous layer was separated, washed with hexanes twice, and the volume reduced under reduced pressure by about half. To this solution was added 3,3',3"-phosphinetriyltripropanoic acid (65 mg) and cyclopropylmethyl bromide (261 mg). The mixture was stirred for 90 minutes at room temperature, then a further 100 mgs of cyclopropylmethyl bromide added. The mixture was stirred overnight, then diluted with ethyl acetate, washed with 1N hydrochloric acid, the ethyl acetate layer separated, dried over sodium sulfate, and the solvent removed under reduced pressure, to provide 3-(cyclopropylmethylthio)-2-(2,2-difluoro-2-(4-fluorophenoxy)ethylamino)propanoic acid (520 mg) as a white solid.

B. Similarly, following the procedure of Example 5A, but replacing 2-(2,2-difluoro-2-(4-fluorophenoxy)ethylamino)-3-(tritylthio)propanoic acid with 2-(2,2-difluoro-2-(4-(methylthio)phenoxy)ethylamino)-3-(tritylthio)propanoic acid, 3-(cyclopropylmethylthio)-2-(2,2-difluoro-2-(4-(methylthio)phenoxy)ethylamino)propanoic acid was prepared.

C. Similarly, following the procedure of Example 5A, but replacing cyclopropylmethyl bromide with 3-(chloromethyl)pyridine, 2-(2,2-difluoro-2-(4-fluorophenoxy)ethylamino)-3-(pyridin-3-ylmethylthio)propanoic acid was prepared.

D. Similarly, following the procedure of Example 4A, but replacing 2-(2,2-difluoro-2-(4-fluorophenoxy)ethylamino)-3-(tritylthio)propanoic acid with with other compounds of formula (6), and optionally replacing cyclopropylmethyl bromide with other compounds of formula R⁸-halo, other compounds of formula (7) are prepared. For example, 3-(cyclopropylmethylthio)-2-(2,2-difluoro-2-(4-(methylsulfonyl)phenoxy)ethylamino)propanoic acid.

### Example 6

### Preparation of a Compound of Formula I

### A. Preparation of a Compound of Formula I in which R¹ is 4-Fluorophenyl, R² is Hydrogen, Y is Oxygen, R³ is Hydrogen, A is Methylene, X is SO₂, R⁸ is Cyclopropylmethyl, E is a Covalent Bond, and Z is-C(R⁵)(R⁶)-R¹⁰, in which R⁵ and R⁶ taken together taken together with the carbon atom to which they are attached are Cyclopropyl and R¹⁰ is Cyano

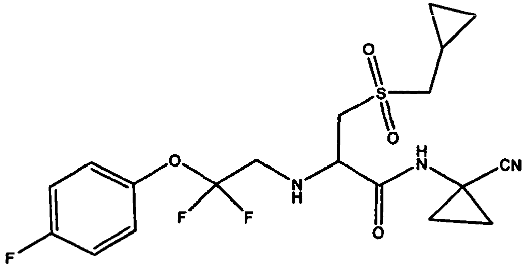

3-(Cyclopropylmethylthio)-2-(2,2-difluoro-2-(4-fluorophenoxy)ethylamino)propanoic acid (120 mg) and N-methylpyrrolidine (4 ml) were stirred with O-(7-azabenzotriazole-1-yl)-N,N,N,N'-tetramethyluronium hexafluorophosphate (259 mg) and 1-aminocyclopropanecarbonitrile (40 mg) at room temperature in the presence of diisopropylethylamine (0.15 ml). After 2 hours Oxone in 4 ml of water was added, and the mixture stirred for 2 hours. The mixture was diluted with ethyl acetate, washed with water, dried over sodium sulfate, and the solvent removed under reduced pressure, to provide a crude product, which was purified by silica gel column chromatography, eluting with ethyl acetate/hexane mixtures, to provide N-(1-cyanocyclopropyl)-3-(cyclopropylmethylsulfonyl) 2-(2,2-difluoro-2-(4-fluorophenoxy)ethylamino)propanamide.

B. Similarly, following the procedure of Example 6A, but replacing 3-(cyclopropylmethylthio)-2-(2,2-difluoro-2-(4-fluorophenoxy)ethylamino)propanoic acid with 2-(2,2-difluoro-2-(4-fluorophenoxy)ethylamino)-3-(pyridin-3-ylmethylthio)propanoic acid, (R)-N-(1-cyanocyclopropyl)-2-(2,2-difluoro-2-(4-fluorophenoxy)ethylamino)-3-(pyridin-3-ylmethylsulfonyl)propanamide was prepared. MS: [M+1]⁺ 483.4.

C. Similarly, following the procedure of Example 6A, but replacing 3-(cyclopropylmethylthio)-2-(2,2-difluoro-2-(4-fluorophenoxy)ethylamino)propanoic acid with 2-(2,2-difluoro-2-(4-(methylthio)phenoxy)ethylamino)-3-(pyridin-3-ylmethylthio)propanoic acid, N-(1-cyanocyclopropyl)-3-(pyridine-3-ylmethylsulfonyl)-2-(2,2-difluoro-2-(4-(methylsulfonyl)phenoxy)ethylamino)propanamide is prepared.

D. Similarly, following the procedure of Example 6A, but replacing 3-(cyclopropylmethylthio)-2-(2,2-difluoro-2-(4-fluorophenoxy)ethylamino)propanoic acid with with other compounds of formula (7), other compounds of Formula I in which R² is hydrogen, R⁴ is -A-X-R⁸, E is a covalent bond, and Z is -C(R⁵)(R⁶)-R¹⁰ are prepared. For example, 3-(cyclopropylmethylthio)-2-(2,2-difluoro-2-(4-(methylsulfonyl)phenoxy)ethylamino)propanoic acid.

### Example 7

### Preparation of a Compound of Formula (10A)

### A. Preparation of a Compound of Formula (10A) in which R¹ is 4-Fluorophenyl, Y is Oxygen, R³ is Hydrogen, A is Methylene, X is Sulfur, R⁸ is Cyclopropylmethyl, R⁵ and R⁶ taken together with the carbon atom to which they are attached are Cyclopropyl, and R¹⁰ is Hydrogen

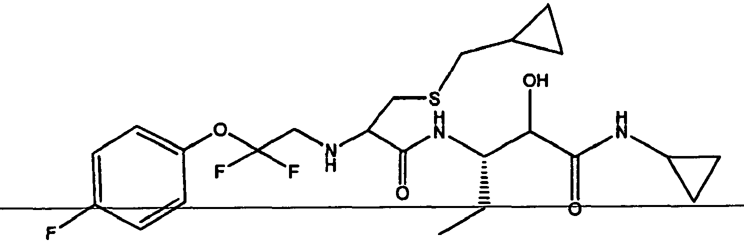

A dichloromethane solution of 3-(cyclopropylmethylthio)-2-(2,2-difluoro-2-(4-fluorophenoxy)ethylamino)-propanoic acid (150 mg), a compound of formula (7), prepared as shown above, (5 ml) (3S)-3-amino-N-cyclopropy-2-hydroxypentanamide, a compound of formula (9), 1-ethyl-3-(3'-dimethylaminopropyl)-carbodimide (122mg), 1-hydroxybenzotriazole (70 mg), and N-methylmorpholine (0.19mL) was stirred at room temperature for 2 hours. The reaction mixture was then diluted with ethyl acetate and washed with aqueous sodium bicarbonate solution, and then dried over anhydrous sodium sulfate. Evaporation to dryness under reduced pressure gave (3S)-N-cyclopropyl-3-(3-(cyclopropylmethylthio)-2-(2,2-difluoro-2-(4-fluorophenoxy)ethylamino)-propanamido)-2-hydroxypentanamide (290 mg).

B. Similarly, following the procedure of Example 7A, but replacing 3-(cyclopropylmethylthio)-2-(2,2-difluoro-2-(4-fluorophenoxy)ethylamino)-propanoic acid with 2-(2,2-difluoro-2-(4-fluorophenoxy)ethylamino)-3-(pyridin-4-ylmethylthio)propanoic acid, (3S)-N-cyclopropyl-3-(3-(pyridine-3-ylmethylthio)-2-(2,2-difluoro-2-(4-fluorophenoxy)ethylamino)-propanamido)-2-hydroxypentanamide was obtained

C. Similarly, following the procedure of Example 7A, but replacing 3-(cyclopropylmethylthio)-2-(2,2-difluoro-2-(4-fluorophenoxy)ethylamino)-propanoic acid with other compounds of formula (7A), and optionally replacing (R)-2-amino-N-cyclopropylbutanamide with other compounds of formula (9), other compounds of formula (10A) are prepared. For example, (3S)-N-cyclopropyl-3-(3-(cyclopropylmethylthio)-2-(2,2-difluoro-2-(4-(methylsulfonyl)phenoxy)ethylamino)-propanamido)-2-hydroxypentanamide.

### Example 8

### Preparation of a Compound of Formula (10B)

### A. Preparation of a Compound of Formula (10B) in which R¹ is 4-Fluorophenyl, Y is Oxygen, R³ is Hydrogen, A is Methylene, X is -SO₂-, R⁸ is Cyclopropylmethyl, R⁵ and R⁶ taken together with the carbon atom to which they are attached are Cyclopropyl, and R¹⁰ is Hydrogen

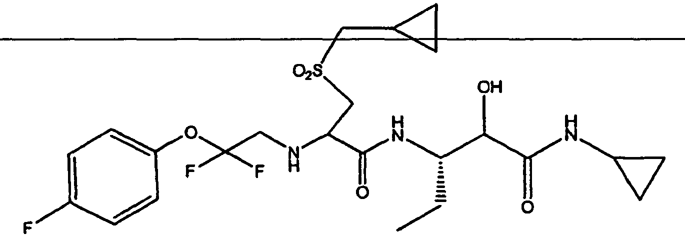

To a solution of (3S)-N-cyclopropyl-3-(3-(cyclopropylmethylthio)-2-(2,2-difluoro-2-(4-fluorophenoxy)ethylamino)-propanamido)-2-hydroxypentanamide (29mg) in N-methylpyrrolidone (5 ml) was added a solution of Oxone (638 mg) in water (5 ml). The mixture was stirred at room temperature for 3 hours, ethyl acetate added, wahed with water x3, dried over sodium sulfate, and solvent removed under reduced pressure, to provide (3S)-N-cyclopropyl-3-(3-(cyclopropylmethylsulfonyl)-2-(2,2-difluoro-2-(4-fluorophenoxy)ethylamino)propanamido)-2-hydroxypentanamide (230 mg).

B. Similarly, following the procedure of Example 8A, but replacing (3S)-N-cyclopropyl-3-(3-(cyclopropylmethylthio)-2-(2,2-difluoro-2-(4-fluorophenoxy)ethylamino)-propanamido)-2-hydroxypentanamide with (3S)-N-cyclopropyl-3-(3-(pyridine-3-ylmethylthio)-2-(2,2-difluoro-2-(4-fluorophenoxy)ethylamino)-propanamido)-2-hydroxypentanamide, (3S)-N-cyclopropyl-3-(3-(pyridine-3-ylmethylsulfonyl)-2-(2,2-difluoro-2-(4-fluorophenoxy)ethylamino)propanamido)-2-hydroxypentanamide was obtained.

C. Similarly, following the procedure of Example 8A, but replacing (3S)-N-cyclopropyl-3-(3-(cyclopropylmethylthio)-2-(2,2-difluoro-2-(4-fluorophenoxy)ethylamino)-propanamido)-2-hydroxypentanamide with other compounds of formula (10A), other compounds of formula (10B) are prepared. For example, (3S)-N-cyclopropyl-3-(3-(cyclopropylmethylsulfonyl)-2-(2,2-difluoro-2-(4-(methylsulfonyl)phenoxy)ethylamino)propanamido)-2-hydroxypentanamide.

### Example 9

### Preparation of a Compound of Formula I

### A. Preparation of a Compound of Formula I in which R¹ is 4-Fluorophenyl, Y is Oxygen, R² and R³ are Hydrogen, A is Methylene, X is -SO₂-, R⁸ is Pyridin-3-ylmethylene, E is - CH(R⁹)-C(O)-C(O)-NH-, in which R⁹ is Ethyl, and Z is -C(R⁵)(R⁶)-R¹⁰, in which R⁵ and R⁶ taken together with the carbon atom to which they are attached are Cyclopropyl, and R¹⁰ is Hydrogen

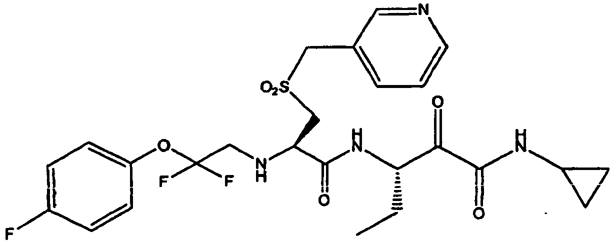

(3S)-N-cyclopropyl-3-(3-(pyridine-3-ylmethylsulfonyl)-2-(2,2-difluoro-2-(4-fluorophenoxy)ethylamino)propanamido)-2-hydroxypentanamide (230 mg) was dissolved in dichloromethane (10 ml) and N-methylpyrrolidine (1.5 ml), and treated with the Dess Martin periodinanane reagent (255 mg) for 1 hour at room temperature. The reaction mixture was then diluted with ethyl acetate, and washed with dilute sodium bicarbonate solution. A solid precipitated out of the solution, and was filtered off, and added to the residue obtained by evaporation of the ethyl acetate layer to dryness under reduced pressure. The product was flash chromatographed on silica gel, eluting with ethyl acetate/hexane1/1, to provide (S)-N-cyclopropyl-3-((R)-2-(2,2-difluoro-2-(4-fluorophenoxy)ethylamino)-3-(pyridin-3-ylmethylsulfonyl)propanamido)-2-oxopentanamide (39 mg). MS: [M+1]⁺ 571.3; NMR (DMSOd₆): δppm; 0.6 (m, 4H), 0.9 (t, 3H), 1.6 (m, 1H), 1.85 (m, 1H), 2.8 (m, 1H), 4.0 (m, 1H), 4.8 (d,d, 2H), 5.0 (m, 1H), 7.25 (m, 4H), 7.45 (m, 1H), 7.8 (m, 1H), 8.7 (m, 4H).

B. Similarly, replacing (3S)-N-cyclopropyl-3-(3-(pyridine-3-ylmethylsulfonyl)-2-(2,2-difluoro-2-(4-fluorophenoxy)ethylamino)propanamido)-2-hydroxypentanamide with (3S)-N-cyclopropyl-3-(3-(cyclopropylmethylsulfonyl)-2-(2,2-difluoro-2-(4-fluorophenoxy)ethylamino)propanamido)-2-hydroxypentanamide, and following the procedures of Example 9A, (S)-N-cyclopropyl-3-((R)-3-(cyclopropylmethylsulfonyl)-2-(2,2-difluoro-2-(4-fluorophenoxy)ethylamino)propanamido)-2-oxopentanamide (31953)was obtained. MS: [M+1]⁺ 534.3; NMR (DMSOd₆): δppm; 0.35 (m, 2H), 0.65 (m, 6H), 0.8 (t, 3H), 1.1 (m, 1H), 1.45 (m, 1H), 1.65 (m, 1H), 2.55 (m, 1H), 3.9 (m, 1H), 4.95 (m, 1H), 7.25 (m, 4H), 8.4 (d, 1H), 8.85 (m, 1H).

C. Similarly, replacing (3S)-N-cyclopropyl-3-(3-(pyridine-3-ylmethylsulfonyl)-2-(2,2-difluoro-2-(4-fluorophenoxy)ethylamino)propanamido)-2-hydroxypentanamide with other compounds of formula (10B), and following the procedures of Example 9A, other compounds of Formula I are prepared. For example, (S)-N-cyclopropyl-3-((R)-2-(2,2-difluoro-2-(4-(methylsulfonyl)phenoxy)ethylamino)-3-(pyridin-3-ylmethylsulfonyl)propanamido)-2-oxopentanamide.

### Example 10

### Preparation of a Compound of Formula (11)

### A. Preparation of a Compound of Formula (11) in which R¹ is 4-Methylthiophenyl, Y is Oxygen, and R⁷ is Methyl

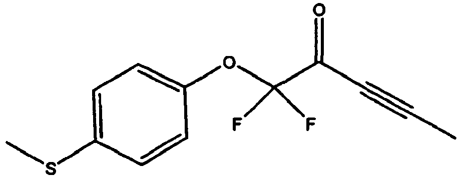

To a solution of prop-1-ynyllithium (0.94 g, prepared by adding n-butyl lithium to prop-1-yne) in tetrahydrofuran at to -78°C was added a solution of ethyl 2,2-difluoro-2-(4-(methylthio)phenoxy)acetate (5.36 g) in tetrahydrofuran and boron trifluoride etherate (2.58 ml, 1 equivalent). The temperature was allowed to warm to -40°C for about 4 hours. Brine was then added to quench the reaction, and the mixture was diluted with ether at room temperature. The organic layer was separated, dried over sodium sulfate, and the solvent removed under reduced pressure, to provide 1,1-difluoro-1-(4-(methylthio)phenoxy)pent-3-yn-2-one.

B. Similarly, following the procedure of Example 10A, but optionally replacing prop-1-ynyllithium with other compounds of formula R⁷-C ≡C-Li, and optionally replacing ethyl 2,2-difluoro-2-(4-(methylthio)phenoxy)acetate with other compounds of formula (2), other compounds of formula (11) are prepared. For example, 1,1-difluoro-1-(4-(methylsulfonyl)phenoxy)pent-3-yn-2-one.

### Example 11

### Preparation of a Compounds of Formula (12)

### A Preparation of a Compound of Formula (12) in which R¹ is 4-Methylthiophenyl, Y is Oxygen, and R⁷ is Methyl

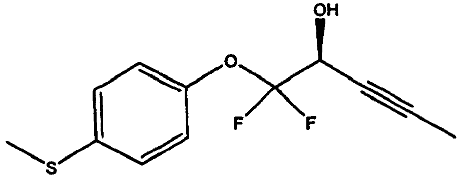

To a solution of 1,1-difluoro-1-(4-(methylthio)phenoxy)pent-3-yn-2-one (1.6 g) in a mixture of dichloromethane (10 ml) and toluene (10 ml) was added Corey catalyst ((S)-1-methyl-3,3-diphenyl-tetrahydro-pyrrolo[1,2-C][1,3,2]oxazaborazole, 0.62 ml). The mixture was cooled to -78°C, and catecholborane (0.89 g) added. The reaction mixture was stirred overnight at -78°C, and then the reaction was quenched by the addition of hydrochloric acid in dioxane and water, followed by aqueous 10% sodium thiosulfate solution. Ether was added, and the organic layer washed with brine, the organic layer separated, solvent removed under reduced pressure, to provide (S)-1,1-difluoro-1-(4-(methylthio)phenoxy)pent-3-yn-2-ol.

B. Similarly, following the procedure of Example 11A, but replacing 1,1-difluoro-1-(4-(methylthio)phenoxy)pent-3-yn-2-one with other compounds of formula (11), other compounds of formula (12) are prepared.

### Example 12

### Preparation of a Compounds of Formula (14)

### A Preparation of a Compound of Formula (14) in which R¹ is 4-Methylthiophenyl, Y is Oxygen, R⁷ is Methyl, R³ is Hydrogen, A is Methylene, X is Sulfur, and Tr is Trityl

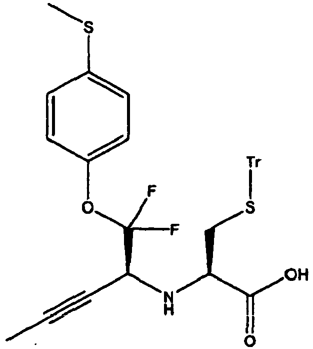

To a solution of (S)-1,1-difluom-1-(4-(methylthio)phenoxy)pent-3-yn-2-ol (1.58 g) in ether at 0°C was added sodium hydride (292 mg of 60%). The mixture was stirred for 1 hour, then trifluoromethanesulfonyl chloride (1.53 g) added. The mixture was stirred for 1 hour, to form the triflate derivative of (S)-1,1-difluoro-1-(4-(methylthio)phenoxy)pent-3-yn-2-ol. This solution was cooled to -78°C, and a mixture of diisopropylethylamine and the trityl derivative of 2-amino-3-mercaptopropanoic acid (1.85 g) in dichloromethane was added dropwise. The reaction mixture was warmed to 0°C, and ethyl acetate added, washed with water, brine, dried over sodium sulfate, and the solvent removed, to provide the trityl derivative of (R)-2-((S)-1,1-difluoro-1-(4-(methylthio)phenoxy)pent-3-yn-2-ylamino)-3-mercaptopropanoic acid.

B. Similarly, following the procedure of Example 12A, but replacing (S)-1,1-difluoro-1-(4-(methylthio)phenoxy)pent-3-yn-2-ol with other compounds of formula (12), other compounds of formula (14) are prepared

### Example 13

### Preparation of a Compound of Formula (15)

### A Preparation of a Compound of Formula (15) in which R¹ is 4-Methylthiophenyl, Y is Oxygen, R⁷ is Methyl, R³ is Hydrogen, A is Methylene, X is Sulfur, and R⁸ is Pyridin-2-ylmethylene

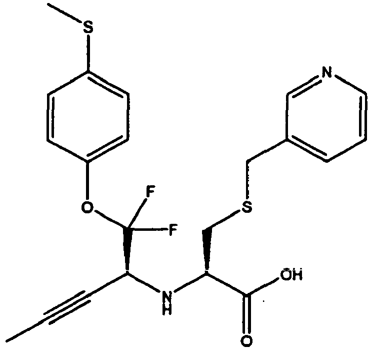

To a solution of the trityl derivative of (R)-2-((S)-1,1-difluoro-1-(4-(methylthio)phenoxy)pent-3-yn-2-ylamino)-3-mercaptopropanoic acid (1.47 g) in dichloromethane (1.5 ml) was added trifluoroacetic acid (0.73 ml) and triethylsilane (0.75 ml). The mixture was then stirred at room temperature for 4 hours, and solvent removed under reduced pressure. To the residue is added hexanes, P(CH₂CH₂CO₂H)₃ (68 mg), and aqueous sodium hydroxide solution (12 ml, 1N), and the mixture extracted with hexanes. To this sodium hydroxide layer was added 3-(chloromethyl)pyridine (125 mg), and the mixture stirred for 2 hours at 40°C. Ethyl acetate was added, the mixture washed with dilute hydrochloric acid, water, brine, and the solvent removed under reduced pressure to provide (R)-2-((S)-1,1-difluoro-1-(4-(methylthio)phenoxy)pent-3-yn-2-ylamino)-3-(pyridin-3-ylmethylthio)propanoic acid. MS: [M+1]⁺ 453.2

B Similarly, following the procedure of Example 13A, but replacing 2 (chloromethyl)pyridine with cyclopropylmethylbromide, (R)-3-(cyclopropylmethylthio)-2-((S)-1,1-difluoro-1-(4-(methylthio)phenoxy)pent-3-yn-2-ylamino)propanoic acid was prepared.

C. Similarly, following the procedure of Example 13A, but optionally replacing (R)-2-((S)-1,1-difluoro-1-(4-(methylthio)phenoxy)pent-3-yn-2-ylamino)-3-mercaptopropanoic acid with other compounds of formula (13), and optionally replacing 2-(chloromethyl)pyridine with other compounds of formula R⁸Halide, other compounds of formula (15) are prepared.

### Example 14

### Preparation of a Compound of Formula I

### A Preparation of a Compound of Formula I in which R¹ is 4-Methylthiophenyl, R² is Prop-1-ynyl, Y is Oxygen, R⁷ is Methyl, R³ is Hydrogen, A is Methylene, X is Sulfur, R⁸ is Pyridin-2-ylmethylene, and Z is -C(R⁵)(R⁶)-R¹⁰; in which R⁵ and R⁶ when taken together with the carbon atom to which they are attached represent Cyclopropyl, and R¹⁰ is Cyano

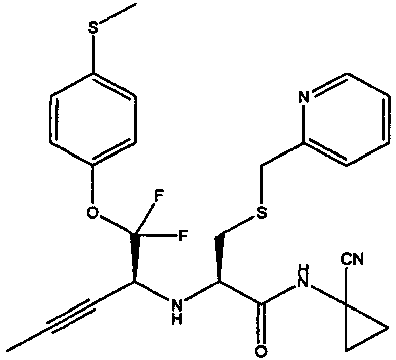

To a stirred mixture of 3-((S)-1,1-difluoro-1-(4-(methylthio)phenoxy)pent-3-yn-2-ylamino)-2-((pyridin-2-yhnethylthio)methyl)propanoic acid (312 mg) and 1-aminocyclopropanecarbonitrile hydrochloride (82 mg) in N,N-dimethylformamide (3 ml), O-(7-azabenzotriazole-1-yl)-N,N,N,N'-tetramethyluronium hexafluorophosphate (259 mg) and diisopropylethylamine (0.36 ml) was added. After stirring overnight at room temperature, the reaction mixture was diluted with ethyl acetate and washed with water, saturated aqueous sodium bicarbonate solution, and brine. After drying over sodium sulfate, the solvent was evaporated and the residue was purified by flash chromatography, eluting with ethyl acetate/hexanes (1/1), to give (R)-N-(1-cyanocyclopropyl)-2-((S)-1,1-difluoro-1-(4-(methylthio)phenoxy)pent-3-yn-2-ylamino)-3-(pyridin-2-ylmethylthio)propanamide (235 mg). MS: [M+1]⁺ 517.3

B. Similarly, following the procedure of Example 14A, but replacing 3-((S)-1,1-difluoro-1-(4-(methylthio)phenoxy)pent-3-yn-2-ylamino)-2-((pyridin-2-ylmethylthio)methyl)propanoic acid with 3-((S)-1,1-difluoro-1-(4-(methylthio)phenoxy)pent-3-yn-2-ylamino)-2-((cyclopropylmethylmethylthio)methyl)propanoic acid, (R)-N-(1-cyanocyclopropyl)-3-(cyclopropylmethylthio)-2-((S)-1,1-difluoro-1-(4-(methylthio)phenoxy)pent-3-yn-2-ylamino)propanamide was prepared.
MS: [M+1]⁺ 480.2

C. Similarly, following the procedure of Example 14A, but replacing 3-((S)-1,1-difluoro-1-(4-(methylthio)phenoxy)pent-3-yn-2-ylamino)-2-((pyridin-2-ylmethylthio)methyl)propanoic acid with 3-((S)-1,1-difluoro-1-(4-(methylthio)phenoxy)pent-3-yn-2-ylamino)-2-((pyridin-3-ylmethylthio)methyl)propanoic acid, (R)-N-(1-cyanocyclopropyl)-3-((S)-1,1-difluoro-1-(4-(methylthio)phenoxy)pent-3-yn-2-ylamino)-2-((pyridin-3-yhnethylthio)methyl)propanamide was prepared.
MS: [M+1]⁺ 517.2

D. Similarly, following the procedure of Example 14A, but optionally replacing 3-((S)-1,1-difluoro-1-(4-(methylthio)phenoxy)pent-3-yn-2-ylamino)-2-((pyridin-2-ylmethylthio)methyl)propanoic acid with other compounds of formula (15), and optionally replacing 1-aminocyclopropanecarbonitrile hydrochloride with other aminonitriles of formula (8) selected from the group consisting of 2-amino-2-methylpropanenitrile, 1-aminocyclobutanecarbonitrile, or 1-aminocyclopentanecarbonitrile, all of which are commercially available, the following compounds are made:

(S)-N-(2-cyanopropan-2-yl)-5-cyclopropyl-2-(2,2-difluoro-2-(4-fluorophenoxy)ethylamino)-4,4-difluoropentanamide;

(25)-N-(1-cyanocyclopropyl)-5-cyclopropyl-2-(1,1-difluoro-1-(4-fluorophenoxy)pent-3-yn-2-ylamino)-4,4-difluoropentanamide;

(2S)-N-(2-cyanopropan-2-yl)-5-cyclopropyl-2-(1,1-difluoro-1-(4-fluorophenoxy)pent-3-yn-2-ylamino)-4,4-difluoropentanamide;

(2S)-N-(1-cyanocyclobutyl)-5-cyclopropyl-2-(1,1-difluoro-1-(4-fluorophenoxy)pent-3-yn-2-ylamino)-4,4-difluoropentanamide; and

(2S)-N-(1-cyanocyclopentyl)-5-cyclopropyl-2-(1,1-difluoro-1-(4-fluorophenoxy)pent-3-yn-2-ylamino)-4,4-difluoropentanamide.

D. Similarly, following the procedure of Example 14A, but replacing 3-((S)-1,1-difluoro-1-(4-(methylthio)phenoxy)pent-3-yn-2-ylamino)-2-((pyridin-2-ylmethylthio)methyl)propanoic acid with other compounds of formula (15), other compounds of Formula I are prepared.

### Example 15

### Preparation of a Compound of Formula I

### A Preparation of a Compound of Formula I in which R¹ is 4-Methylsulfonylphenyl, R² is Prop-1-ynyl, Y is Oxygen, R⁷ is Methyl, R³ is Hydrogen, A is Methylene, X is -SO₂-, R⁸ is Pyridin-2-ylmethylene, and Z is -C(R⁵)(R⁶)-R¹⁰; in which R⁵ and R⁶ when taken together with the carbon atom to which they are attached represent Cyclopropyl, and R¹⁰ is Cyano

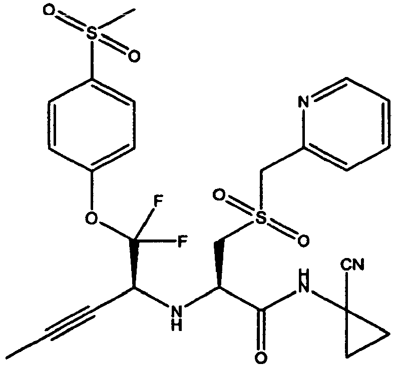

A solution of (R)-N-(1-cyanocyclopropyl)-2-((S)-1,1-difluoro-1-(4-(methylthio)phenoxy)pent-3-yn-2-ylamino)-3-(pyridin-2-ylmethylthio)propanamide (235 mg) in N-methylpyrrolidine (5.5 ml) was added a solution of Oxone (905 mg) in water (5 ml). After stirring for 3 hours at 45 °C the solvent was evaporated under reduced pressure, and the residue was partitioned between dichloromethane (15 ml) and water (15 ml). The organic phase was separated, and the aqueous solution was extracted with dichloromethane. The combined organic layers were washed with brine and dried over sodium sulfate. After removal of the solvent under reduced pressure, the crude was purified by preparative TLC, using ethyl acetate/hexanes (1/1) as mobile phase to give (R)-N-(1-cyanocyclopropyl)-2-((S)-1,1-difluoro-1-(4-(methylsulfonyl)phenoxy)pent-3-yn-2-ylamino)-3-(pyridin-2-ylmethylsulfonyl)propanamide (105 mg). MS: [M+1]⁺ 581.2

B. Similarly, following the procedure of Example 15A, but replacing 1-((R)-3-((S)-1,1-difluoro-1-(4-(methylthio)-phenoxy)pent-3-yn-2-ylamino)-2-oxo-4-(pyridin-2-ylmethylthio)butyl)cyclopropanecarbonitrile with (R)-N-(1-cyanocyclopropyl)-3-(cyclopropylmethylthio)-2-((S)-1,1-difluoro-1-(4-(methylthio)phenoxy)pent-3-yn-2-ylamino)propanamide, (R)-N-(1-cyanocyclopropyl)-3-(cyclopropylmethylsulfonyl)-2-((S)-1,1-difluoro-1-(4-(methylsulfonyl)phenoxy)pent-3-yn-2-ylamino)propanamide was prepared. MS: [M+1]⁺ 544.3

C. Similarly, following the procedure of Example 15A, but replacing 1-((R)-3-((S)-1,1-difluoro-1-(4-(methylthio)-phenoxy)pent-3-yn-2-ylamino)-2-oxo-4-(pyridin-2-ylmethylthio)butyl)cyclopropanecarbonitrile with (R)-N-(1-cyanocyclopropyl)-3-((S)-1,1-difluoro-1-(4-(methylthio)phenoxy)pent-3-yn-2-ylamino)-2-((Pyridin-3-ylmethylthio)methyl)propanamide, 1-((R)-3-((S)-1,1-difluoro-1-(4-(methylsulfonyl)phenoxy)pent-3-yn-2-ylamino)-2-oxo-4-(pyridin-3-ylmethylsulfonyl)butyl)cyclopropanecarbonitrile was prepared. MS: [M+1]⁺ 581.1

D. Similarly, following the procedure of Example 15A, but replacing N-(1-cyanocyclopropyl)-3-((S)-1,1-difluoro-1-(4-(methylthio)phenoxy)pent-3-yn-2-ylamino)-2-((cyclopropylmethylthio)methyl)-propanamide with other compouynds of Formula I in which X is sulfur, other compounds of Formula I in which X is -SO₂ are prepared. For example:

E. (2S)-N-(1-cyanocyclopropyl)-5-cyclopropyl-2-(1,1-difluoro-1-(4-(methylsulfonyl)phenoxy)pent-3-yn-2-ylamino)-4,4-difluoropentanamide.

### Example 16

### Preparation of 1-aminocyclopropanecarbonitrile hydrochloride

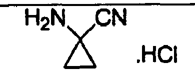

### Step 1

A mixture ofbenzophenone imine (25 g, 0.138 mol, Aldrich) and aminoacetonitrile hydrochloride (25 g, 0.270 mol, Lancaster) in dichloromethane (1000 ml) was stirred in a 2L Erlenmeyer flask under nitrogen at room temperature for 5 days. The reaction mixture was filtered to remove the precipitated ammonium chloride and the filtrate was evaporated to dryness *in vacuo.* The resulting residue was dissolved in ether (400 ml) washed with water (200 ml) and brine. After drying over magnesium sulfate the solution was evaporated to give (benzhydrylideneamino)-acetonitrile (47.89 g).

### Step 2

A solution of sodium hydroxide (91 g, 2.275 mol) in water (91 ml) in a 2L flask was cooled on ice under nitrogen and then treated with benzyl triethyl ammonium chloride (2.0 g, 0.0088 mol, Aldrich ) and (benzhydrylideneamino)acetonitrile (47.89 g) in toluene (100 ml). 1,2-Dibromoethane (23 ml, 122.4 mmol, Aldrich) was then added dropwise over 25 min, to the reaction mixture with mechanical stirring and cooling to maintain the internal temperature near +10°C. The reaction mixture was then stirred vigorously for 24 h at room temperature and then poured into ice water and extracted with toluene. The combined extracts were washed with brine and then treated with MgSO4 and Norite. After filtering, toluene was removed by rotary evaporation to give an oil (67 g). The residue was dissolved in boiling hexane (400 ml), treated with Norite and filtered hot and allowed to cool. A dark oil separated and which was removed by pipet (~2 ml). Scratching induced crystallization in the remaining solution which was cooled on ice for 2 h. Light yellow crystals were collected by filtration and washed with cold hexane to give 1-(benzhydrylideneamino)cyclopropanecarbonitrile (30.56 g).

### Step 3

A mixture of 1-(benzhydrylideneamino)cyclopropanecarbonitrile (30.56 g, 0.124 mol) in concentrated HCl (12 ml) in water (100 ml) and ether (100 ml) was stirred at room temperature for 15 h. The ether layer was discarded and the aqueous layer was washed with ether. The aqueous layer was then freeze dried to give the title compound as a tan powder (13.51 g).

### Example 17

### Preparation of (R)-methyl 2-(tert-butoxycarbonylamino)-3-iodopropanoate (17)

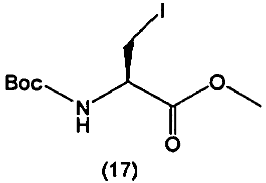

Triphenylphosphite methiodide (Fieser and Fieser, Reagents for Organic Synthesis, Vol. 4, p557; 340g, 753 mmol, 1.4 equiv.) was added in one portion to a solution of (*S*)-2-*tert*-butyloxycarbonylamino-3-hydroxypropionic acid methyl ester (118g, 538 mmol) in dry N,N-dimethylformamide (1.1L) at 0°C. After 30 minutes at 0°C solid sodium bicarbonate (270g) was added followed by water (1.1L). The resulting mixture was stirred vigorously for 15 minutes and then extracted with 1:1 diethylether/hexanes (3 x 800mL). The combined organic extracts were washed with 0.5M sodium hydroxide (5 x 1L) and brine (2 x 1L), dried over magnesium sulfate, filtered, and the filtrate concentrated under reduced pressure. The resulting orange oil was loaded onto a SiO₂ plug (2" x 7") and eluted with 5% EtOAc/hexanes to afford (R)-methyl 2-(tert-butoxycarbonylamino)-3-iodopropanoate as a pinkish oil, which solidified (118.5g, 67%) ¹H NMR (CDC13; 400MHz): δppm 5.32 (m, 1H), 4.51 (m, 1H), 3.81 (s, 3H), 3.58 (m, 2H), 1.45 (s, 9H).

### Example 18

### Preparation of (R)-(2-(tert-butoxycarbonylamino)-3-methoxy-3-oxopropyl)zinc(II) iodide (18)

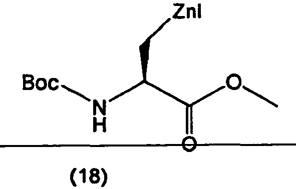

Zinc dust (<10 micron, 94g, 1.44mol), 4 equiv.) was suspended in 2M hydrochloric acid and stirred vigorously for 5 minutes. After filtration the Zn was sequentially washed with water, ethanol, and diethylether. This material was transferred to a 3L flask, placed under high vacuum (0. 1mmHg) and heated externally with a heat gun for several minutes, then allowed to cool to room temperature. The heated/cooling procedure (under vacuum) was repeated twice more, and then the vessel was purged with nitrogen.

The zinc was suspended in dry benzene (1.44L) and dry N,N-dimethylacetamide (96mL) and treated with 1,2-dibromoethane (3.1mL, 10mol%). This mixture was subject to gentle external heating until the formation of small bubbles of gas was observed, and this procedure repeated several times over 1 hour. Trimethylsilylchloride (2.3mL, 5mol%) was then added and the mixture stirred at room temperature for 30 minutes.

To this mixture of activated zinc was added dropwise a solution of (*R*)-2-*tert-*butyloxycarbonylamino-3-iodopropionic acid methyl ester (118.5g, 360mmol) in dry benzene (144mL) and dry N,N-dimethylacetamide (10mL). On complete addition the reaction was stirred for 2 hours, treated with a further portion of trimethylsilyl chloride (2.3mL), and stirred for an additional 30minutes. This product of formula (18), (R)-(2-(tert-butoxycarbonylamino)-3-methoxy-3-oxopropyl)zinc(II) iodide, was used in the next step with no further purification.

### Example 19

### Preparation of (S)-methvl 2-(tert-butoxycarbonylamino)-5-cyclopropyl-4-oxopentanoate (20)

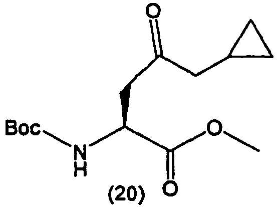

A. To the mixture obtained in Example 18 (the compound of formula (18)) PdCl₂(PPh₃)₂ (12.6g, 5mol%) was added in a single portion, followed by dropwise addition of a solution of cyclopropylacetyl chloride (1 equiv.) in dry benzene (144mL). On complete addition the reaction mixture was stirred for 45 minutes, then treated with 2M hydrochloric acid (1.7L), stirred for 10 minutes, then filtered through celite, eluting with diethylether (1.7L). The layers were separated, the aqueous layer extracted with diethylether (1.25L), and the combined organic extracts washed with water (1.25L), brine (1.25L), dried over magnesium sulfate, filtered, and the filtrate concentrated under reduced pressure.

Purification of the residue by column chromatography (SiO₂, gradient elution 0% to 20% ethyl aeetate/Hexanes, 10% steps) gave (S)-methyl 2-(tert-butoxycarbonylamino)-5-cyclopropyl-4-oxopentanoate (R_{f} 0.18; 25% EtOAc/hexanes) as a dark oil, which also contained some residual metal solids that was removed by filtration of an Et₂O solution through celite (85.5g, 83%).

¹H NMR (CDCl3; 400MHz): δppm 5.43 (m, 1H); 4.43 (m, 1H), 3.68 (s, 3H), 3.15 (m, 1H), 2.92 (m, 1H), 2.22 (m, 2H), 1.40 (s, 9H), 0.88 (m, 1H), 0.5 (m, 2H), 0.08 (m, 2H).

B. Similarly, following the procedure of Example 19A, replacing cyclopropylacetyl chloride with 2-phenylacetyl chloride, (S)-methyl 2-(tert-butoxycarbonylamino)-4-oxo-5-phenylpentanoate is prepared.

Similarly, replacing cyclopropylacetyl chloride with 2-(4-fluorophenyl)acetyl chloride, (S)-methyl 2-(tert-butoxycarbonylamino)-5-(4-fluorophenyl)-4-oxopentanoate is prepared.

Similarly, replacing cyclopropylacetyl chloride with 2-(pyridin-3-yl)acetyl chloride, (S)-methyl 2-(tert-butoxycarbonylamino)-4-oxo-5-(pyridin-3-yl)pentanoate is prepared.

### Example 20

### Preparation of (S)-methyl 2-(tert-butoxycarbonylamino)-5-cyclopropyl-4,4-difluoropentanoate (21)

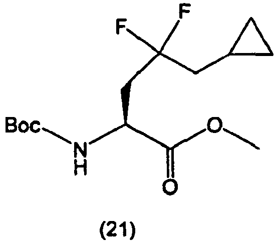

A. To neat (S)-methyl 2-(tert-butoxycarbonylamino)-5-cyclopropyl-4-oxopentanoate (53g, 185 mmol) was added diethylaminosulfur trifluoride (DAST) (48mL, 370 mmol, 2 equiv.) and the resulting dark, viscous mixture stirred at room temperature for approximately 135 hours. After dilution with dichloromethane (500mL) the mixture was poured into 2M sodium hydroxide (1L), the aqueous phase separated, and extracted with methylene chloride (300mL). The combined organic extracts were washed with water (500mL), brine (500mL), dried over magnesium sulfate, filtered, and the filtrate concentrated under reduced pressure. The residue was purified by column chromatography to give (S)-methyl 2-(tert-butoxycarbonylamino)-5-cyclopropyl-4,4-difluoropentanoate as a dark oil (19.3g, 34%). Unreacted starting material (17.5g, 33%) was also recovered. ¹H NMR (CDCl3; 400MHz): δppm 5.02 (m, 1H); 4.36 (m, 1H), 3.59 (s, 3H), 2.28 (m, 2H), 1.62 (m, 1H), 2.22 (m, 2H), 1.27 (s, 9H), 0.65 (m, 1H), 0.38 (m, 2H), 0.02 (m, 2H).

B. Similarly, following the procedure of Example 20A, replacing (S)-methyl 2-(tert-butoxycarbonylamino)-5-cyclopropyl-4-oxopentanoate with (S)-methyl 2-(tert-butoxycarbonylamino)-4-oxo-5-phenylpentanoate, (S)-methyl 2-(tert-butoxycarbonylamino)-5-(4-fluorophenyl)-4-oxopentanoate, or (S)-methyl 2-(tert-butoxycarbonylamino)-4-oxo-5-(pyridin-3-yl)pentanoate, the following compounds of formula (21) are prepared:
(S)-methyl 2-(tert-butoxycarbonylamino)-4,4-difluoro-5-phenylpentanoate;
(S)-methyl 2-(tert-butoxycarbonylamino)-4,4-difluoro-5-(4-fluorophenyl)pentanoate; and
(S)-methyl 2-(tert-butoxycarbonylamino)-4,4-difluoro-5-(pyridin-3-yl)pentanoate.

### Example 21

### Preparation of (S)-2-amino-5-cyclopropyl-4,4-difluoropentanoic acid hydrochloride (22)

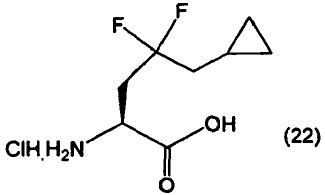

A. To (S)-methyl 2-(tert-butoxycarbonylamino)-5-cyclopropyl-4,4-difluoropentanoate (2.0g, 6.507mmol) in water (20ml) and tetrahydrofuran (5ml) was added lithium hydroxide (0.47g, 19.51mmol), and the mixture stirred overnight at ambient temperature. The product was acidified with citric acid (5g), extracted into ethyl acetate ( 250ml), and the organic layer separated, dried over magnesium sulfate, and filtered through a small pad of silica. The solvent was evaporated under reduced pressure to yield 1.6g of an orange oil. This oil was dissolved in dichloromethene (5ml) and 4M hydrochloric acid in dioxane (16.25ml) was added, and the mixture stirred for 4 hours. The solvents were evaporated to dryness, and the resulting solid triturated with diethylether to give 1.07g( 71.8%) of (S)-2-amino-5-cyclopropyl-4,4-difluoropentanoic acid hydrochloride as a buff colored solid.

¹H NMR (CDCl3; 400MHz): δppm 8.42 (bs, 3H); 4.08 (m, 1H), 3.25 (bs, 1H), 2.58 (m, 2H), 1.82 (m, 2H), 1.4 (1H), 0.75 (m, 1H), 0.48 (m, 2H), 0.18 (m, 2H).

B. Similarly, following the procedure of Example 21A, replacing (S)-methyl 2-(tert-butoxycarbonylamino)-5-cyclopropyl-4,4-difluoropentanoate with (S)-methyl 2-(tert-butoxycarbonylamino)-4,4-difluoro-5-phenylpentanoate, (S)-2-amino-4,4-difluoro-5-phenylpentanoic acid hydrochloride is prepared.

Similarly, following the procedure of Example 21A, replacing (S)-methyl 2-(tert-butoxycarbonylamino)-5-cyclopropyl-4,4-difluoropentanoate with (S)-methyl 2-(tert-butoxycarbonylamino)-4,4-difluoro-5-(4-fluorophenyl)pentanoate, (S)-2-amino-4,4-difluoro-5-(4-fluorophenyl)pentanoic acid hydrochloride is prepared.

Similarly, following the procedure of Example 21A, replacing (S)-methyl 2-(tert-butoxycarbonylamino)-5-cyclopropyl-4,4-difluoropentanoate with (S)-methyl 2-(tert-butoxycarbonylamino)-4,4-difluoro-5-(pyridin-3-yl)pentanoate, (S)-2-amino-4,4-difluoro-5-(pyridin-3-yl)pentanoic acid hydrochloride is prepared.

### Example 22

### Preparation of a Compound of Formula (23)

### Preparation of a Compound of Formula (23) in which R¹ is 4-Fluorophenyl and Y is Oxygen

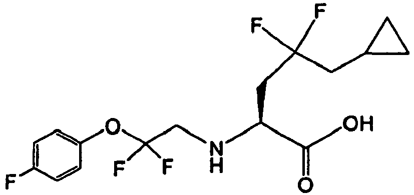

A. 1,1-Difluoro-1-(4-fluorophenoxy)-ethanol (0.84g, 4.35mmol) and diisopropylethylamine (0.84g, 6.53mmol, 1.5eq) was dissolved in dichloromethane (10ml)), and added to a solution of triflic anhydride (1.29g , 4.57mmol, 1.05eq) in dichloromethane (15ml) at -78°C. The mixture was stirred for 15 minutes, and then allowed to warm to room temperature. To this product was added (S)-2-amino-5-cyclopropyl-4,4-difluoropentanoic acid hydrochloride (1.00g, 4.35mmol) and diisopropylethylamine (2.25g, 17.40mmol, 4.0eq) and the mixture heated to 40°C for 48 hours. The solvent was evaporated under reduced pressure, and the residue partitioned between aqueous citric acid (10.0g in 250ml water) and ethyl acetate (250ml). The organic layer was separated and dried over magnesium sulfate, and the solvent evaporated from the filtrate under reduced pressure to give (S)-5-cyclopropyl-2-(2,2-difluoro-2-(4-fluorophenoxy)ethylamino)-4,4-difluoropentanoic acid as an off-white solid, which was washed with diisopropyl ether, filtered off, and dried. Yield 410mg (25.6%) ¹H NMR (CDCl3; 400MHz): δppm 7.22 (m, 4H); 3.52-3.05 (m, 3H), 2.48 (m, 2H), 1.91 (m, 2H), 1.24 (m, 1H), 0.8 (m, 1H), 0.46 (m, 2H), 0.12 (m, 2H). LC/MS: retention time; t = 3.24 min Mass: (M+H)⁺ 368; Mass: (M-H)- 366.

### B. Preparation of other Compounds of Formula (23), varying R¹ and Y

Similarly, following the procedure of Example 22A, but replacing 1,1-difluoro-1-(4-fluorophenoxy)-ethanol with (S)-2-amino-4,4-difluoro-5-phenylpentanoic acid hydrochloride, (S)-2-(2,2-difluoro-2-(4-fluorophenoxy)ethylamino)-4,4-difluoro-5-phenylpentanoic acid is prepared.

Similarly, following the procedure of Example 22A, but replacing 1,1-difluoro-1-(4-fluorophenoxy)-ethanol with (S)-2-amino-4,4-difluoro-5-(4-fluorophenyl)pentanoic acid hydrochloride, (S)-2-(2,2-difluoro-2-(4-fluorophenoxy)ethylamino)-4,4-difluoro-5-(4-fluorophenyl)pentanoic acid is prepared.

Similarly, following the procedure of Example 22A, but replacing 1,1-difluoro-1-(4-fluorophenoxy)-ethanol with (S)-2-amino-4,4-difluoro-5-(pyridin-3-yl)pentanoic acid hydrochloride, (S)-2-(2,2-difluoro-2-(4-fluorophenoxy)ethylamino-4,4-difluoro-5-(pyridin-3-yl)pentanoic acid is prepared.

### C. Preparation of a Compound of Formula (23), varying R¹ and Y

Similarly, following the procedure of Example 22A, but optionally replacing 1,1-difluoro-1-(4-fluorophenoxy)-ethanol with other compounds of formula (4), other compounds of formula (23) are prepared.

### Example 23

### Preparation of a Compound of Formula I

### Preparation of a Compound of Formula I in which R¹ is 4-Fluorophenyl, Y is Oxygen, R² and R³ are Hydrogen, R⁴ is -A-X-R⁸, in which A is -CH₂-CF₂, X is a Covalent Bond, and R⁸ is Cyclopropylmethyl, E is a Covalent Bond, and Z is 1-Cyanocyclopropyl

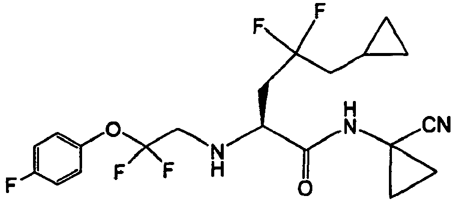

A. To a solution of (S)-5-cyclopropyl-2-(2,2-difluoro-2-(4-fluorophenoxy)ethylamino)-4,4-difluoropentanoic acid (200mg, 0.54mmol) and 1-aminocyclopropanecarbonitrile hydrochloride (65mg,0.54mmol) in dichloromethane was added O-(7-azabenzotriazole-1-yl)-N,N,N,N'-tetramethyluronium hexafluorophosphate (HATU) (228mg, 0.60mmol, 1.1eq), and the mixture was stirred for 5 minutes. N-Methylmorpholine (220mg,2.18mmol,4.0eq) was them added, and stirred at room temperature overnight. To the reaction mixture was added aqueous potassium carbonate (200ml)) and the product was extracted into ethyl acetate (200ml)). The organic layer was dried over magnesium sulate, filtered through a small pad of silica, and the filtrate evaporated under reduced pressure. The residue was chromatographed on silica gel, eluting with 2:1 hexane/ethyl acetate. The product was isolated as 100mg of a yellow oil. Trituration with hexane afforded 84mg (36%) of (S)-N-(1-cyanocyclopropyl)-5-cyclopropyl-2-(2,2-difluoro-2-(4-fluorophenoxy)ethylamino)-4,4-difluoropentanamide as a white solid.
¹H NMR (d6-DMSO; 400MHz): δppm 8.97 (s, 1H), 7.25 (m, 4H), 3.41 (m, 1H), 3.20 (m, 1H), 3.05 (m, 1H), 2.70 (m, 1H), 2.25 (m, 2H), 1.85 (m, 2H), 1.47 (m, 2H), 1.22 (m, 2H), 0.8 (m, 1H), 0.45 (m, 2H), 0.12 (m, 2H). LC/MS: retention time; t = 4.53 min Mass: (M+H)⁺ 432; Mass: (M-H)⁻ 430.

### B. Preparation of other Compounds of Formula I in which R² and R³ are Hydrogen, R⁴ is -A-X-R⁸, in which A is -CH₂-CF₂, X is a Covalent Bond, R⁸ is Cyclopropylmethyl, E is a Covalent Bond, and Z is 1-Cyanocyclopropyl, varying R¹ and Y

Similarly, following the procedure of Example 23A, but replacing 1-aminocyclopropanecarbonitrile hydrochloride with a compound of formula (8) selected from 2-amino-2-methylpropanenitrile, 1-aminocyclobutanecarbonitrile, and 1-aminocyclopentanecarbonitrile, all of which are commercially available, and optionally replacing (S)-5-cyclopropyl-2-(2,2-difluoro-2-(4-fluorophenoxy)ethylamino)-4,4-difluoropentanoic acid with other compounds of formula (23), the following compounds are made:

(S)-N-(2-cyanopropan-2-yl)-5-cyclopropyl-2-(2,2-difluoro-2-(4-fluorophenoxy)ethylamino)-4,4-difluoropentanamide;

(S)-N-(2-cyanopropan-2-yl)-5-cyclopropyl-2-(2,2-difluoro-2-(4-(methylthio)phenoxy)ethylamino)-4,4-difluoropentanamide ;

(S)-N-(1-cyanocyclobutyl)-5-cyclopropyl-2-(2,2-difluoro-2-(4-fluorophenoxy)ethylamino)-4,4-difluoropentanamide;

(S)-N-(1-cyanocyclobutyl)-5-cyclopropyl-2-(2,2-difluoro-2-(4-fluorophenoxy)ethylamino)4,4-difluoropentanamide;

(S)-N-(1-cyanocyclopentyl)-5-cyclopropyl-2-(2,2-difluoro-2-(4-methylthiophenoxy)ethylamino)-4,4-difluoropentanamide;

(S)-N-cyclopropyl-3-((S)-5-cyclopropyl-2-(2,2-difluoro-2-(4-fluorophenoxy)ethylamino)-4,4-difluoropentanamido)-2-oxohexanamide;

(S)-N-cyclopropyl-3-((S)-5-cyclopropyl-2-(2,2-difluoro-2-(4-methylthiophenoxy)ethylamino)-4,4-difluoropentanamido)-2-oxohexanamide;

(S)-N-((S)-1-cyclobutyl-4-(cyclopropylamino)-3,4-dioxobutan-2-yl)-5-cyclopropyl-2-(2,2-difluoro-2-(4-fluorophenoxy)ethylamino)-4,4-difluoropentanamide;

(S)-N-((S)-1-cyclobutyl-4-(cyclopropylamino)-3,4-dioxobutan-2-yl)-5-cyclopropyl-2-(2,2-difluoro-2-(4-methylthiophenoxy)ethylamino)-4,4-difluoropentanamide.

(S)-N-(1-cyanocyclopropyl)-2-(2,2-difluoro-2-(4-fluorophenoxy)ethylamino)-4,4-difluoro-5-phenylpentanamide;

(S)-N-(1-cyanocyclopropyl)-2-(2,2-difluoro-2-(4-methylthiophenoxy)ethylamino)-4,4-difluoro-5-phenylpentanamide

(S)-N-(1-cyanocyclopropyl)-2-(2,2-difluoro-2-(4-fluorophenoxy)ethylamino)-4,4-difluoro-5-(4-fluorophenyl)pentanamide;

(S)-N-(1-cyanocyclopropyl)-2-(2,2-difluoro-2-(4-methylthiophenoxy)ethylamino)-4,4-difluoro-5-(4-fluorophenyl)pentanamide;

(S)-N-(1-cyanocyclopropyl)-2-(2,2-difluoro-2-(4-fluorophenoxy)ethylamino)-4,4-difluoro-5-(pyridin-3-yl)pentanamide;

(S)-N-(1-cyanocyclopropyl)-2-(2,2-difluoro-2-(4-methylthiophenoxy)ethylamino)-4,4-difluoro-5-(pyridin-3-yl)pentanamide;

(S)-N-(2-cyanopropan-2-yl)-2-(2,2-difluoro-2-(4-fluorophenoxy)ethylamino)-4,4-difluoro-5-(4-fluorophenyl)pentanamide;

(S)-N-(1-cyanocyclobutyl)-2-(2,2-difluoro-2-(4-fluorophenoxy)ethylamino)-4,4-difluoro-5-(4-fluorophenyl)pentanamide; and

(S)-N-(1-cyanocyclopentyl)-2-(2,2-difluoro-2-(4-fluorophenoxy)ethylamino)-4,4-difluoro-5-(4-fluorophenyl)pentanamide.

### C. Preparation of other Compounds of Formula I in which R² and R³ are Hydrogen, R⁴ is -A-X-R⁸, in which A is -CH₂CF₂, X is a Covalent Bond, R⁸ is Cyclopropylmethyl, and E is a Covalent Bond, and Z is 1-Cyanocyclopropyl, varying R¹ and Y

Similarly, following the procedure of Example 23A, but replacing (S)-5-cyclopropyl-2-(2,2-difluoro-2-(4-fluorophenoxy)ethylamino)-4,4-difluoropentanoic acid with other compounds of formula (23), other compounds of Formula I are prepared.

### Example 24

### Preparation of a Compound of Formula (24)

### Preparation of a Compound of Formula (24) in which R¹ is 4-Fluorophenyl and Y is Oxygen

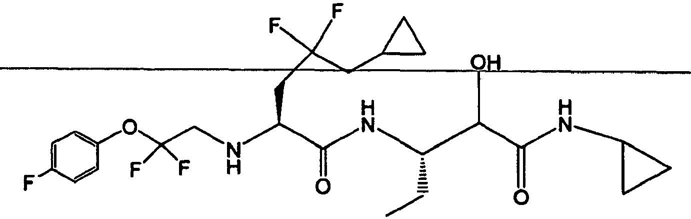

A. To a mixture of (S)-5-cyclopropyl-2-(2,2-difluoro-2-(4-fluorophenoxy)ethylamino)-4,4-difluoropentanoic acid (200mg, 0.54mmol) and (3S)-3-amino-N-cyclopropyl-2-hydroxypentanamide hydrochloride (114mg, 0.54mmol) in dichloromethane (12ml) was added O-(7-azabenzotriazole-1-yl)-N,N,N,N'-tetramethyluronium hexafluorophosphate (HATU, (228mg, 0.60mmol, 1.1eq), and the mixture was stirred for 5 minutes before the addition of N-methylmorpholine (220mg, 218mmol, 4.0eq). After stirring overnight at room temperature the product was partitioned between aqueous citric acid (10g in 250ml) and ethyl acetate (250ml). The organic layer was separated, washed with brine, dried over magnesium sulphate, filtered, and solvent evaporated from the filtrate to give 200mg of (2S)-5-cyclopropyl-N-((3S)-1-(cyclopropylamino)-2-hydroxy-1-oxopentan-3-yl)-2-(2,2-difluoro-2-(4-fluorophenoxy)ethylamino)-4,4-difluoropentanamide as a pale yellow semi-solid.
¹H NMR (d6-DMSO; 400MHz): δppm 7.82-7.60 (m, 2H), 7.42 (m, 4H), 5.58 (m, 1H), 3.95 (m, 1H), 3.85 (m, 1H), 3.44 (m, 1H), 3.20 (m, 1H), 3.00 (m, 1H), 2.62 (m, 2H), 2.35 -2.00 (m, 2H) 1.90 (m, 2H), 1.55 (m, 1H), 1.35 (m), 0.8 (m, 4H), 0.60-0.40 (m, 6H), 0.12 (m, 2H). LC/MS: retention time; t = 4.36 min Mass: (M+H)⁺ 522; Mass: (M-H)⁻ 520.

### B. Preparation of Compounds of Formula (24), varying R¹ and Y

Similarly, following the procedure of Example 24A, but replacing (S)-5-cyclopropyl-2-(2,2-difluoro-2-(4-fluorophenoxy)ethylamino)-4,4-difluoropentanoic acid with other compounds of formula (23), other compounds of formula (24) are prepared.

### Example 25

### Preparation of a Compound of Formula I

### A. Preparation of a Compound of Formula I in which R¹ is 4-Fluorophenyl, Y is Oxygen, R² and R³ are Hydrogen, R⁴ is -A-X-R⁸, in which A is -CH₂-CF₂, X is a Covalent Bond, R⁸ is Cyclopropylmethyl, E is -CH(R⁹)-C(O)-C(O)-NH-, in which R⁹ is Ethyl, and R⁵ and R⁶ taken together are Cyclopropyl and R¹⁰ is Hydrogen

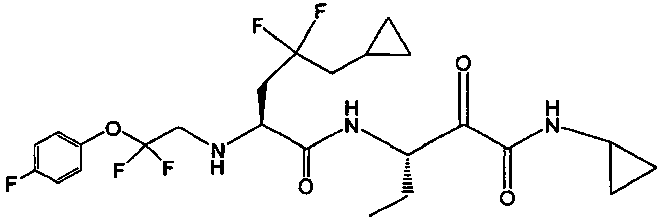

To a solution of (2S)-5-cyclopropyl-N-((3S)-1-(cyclopropylamino)-2-hydroxy-1-oxopentan-3-yl)-2-(2,2-difluoro-2-(4-fluorophenoxy)ethylamino)-4,4-difluoropentanamide (190mg, 0.36mmol) in N-methylpyrrolidine (4ml) was added Dess-Martin periodinane reaebt (216mg, 0.51mmol, 1.4eq). The mixture was stirred for 4 hours at room temperature, then added to saturated aqueous sodium bicarbonate (50ml) containing sodium thiosulfate (1.5g). The mixture was stirred for 1hour, filtered, the solid material washed with water, then dried in a vacuum oven at 40°C using phosphorus pentoxide as a drying agent, yielding (S)-5-cyclopropyl-N-((S)-1-(cyclopropylamino)-1,2-dioxopentan-3-yl)-2-(2,2-difluoro-2-(4-fluorophenoxy)ethylamino)-4,4-difluoropentanamide 144mg (76%)

¹H NMR (d6-DMSO; 400MHz): δppm 8.65 (1H), 8.38 (1H), 7.35 (m, 4H), 4.90 (m, 1H), 3.48 (m, 1H), 3.22 (m, 1H), 3.05 (m, 1H), 2.72 (m, 1H), 2.65 (m, 1H), 2.35 -2.00 (m), 1.55 (m, 1H), 1.35 (m), 0.90 - 0.42 (m, 10H), 0.60 - 0.40 (m, 6H), 0.12 (m, 2H). LC/MS: retention time; t = 20.01 min Mass: (M+H)⁺ 520.

### B: Preparation of Compounds of Formula I varying R¹and Y

Similarly, following the procedure of Example 25A, but replacing (2S)-5-cyclopropyl-N-((3S)-1-(cyclopropylamino)-2-hydroxy-1-oxopentan-3-yl)-2-(2,2-difluoro-2-(4-fluorophenoxy)ethylamino)-4,4-difluoropentanamide with other compounds of formula (24), other compounds of Formula I are prepared. For example:

(S)-N-cyclopropyl-3-((S)-5-cyclopropyl-2-(2,2-difluoro-2-(4-fluorophenoxy)ethylamino)-4,4-difluoropentanamido)-2-oxohexanamide;

(S)-N-((S)-1-cyclobutyl-4-(cyclopropylamino)-3,4-dioxobutan-2-yl)-5-cyclopropyl-2-(2,2-difluoro-2-(4-fluorophenoxy)ethylamino)-4,4-difluoropentanamide;

(S)-N-cyclopropyl-3-((S)-2-(2,2-difluoro-2-(4-fluorophenoxy)ethylamino)-4,4-difluoro-5-(4-fluorophenyl)pentanamido)-2-oxohexanamide; and

(S)-N-((S)-1-(cyclopropylamino)-1,2-dioxopentan-3-yl)-2-(2,2-difluoro-2-(4-fluorophenoxy)ethylamino)-4,4-difluoro-5-(4-fluorophenyl)pentanamide.

### Biological Examples

### EXAMPLE 1

### Cathepsin B Assay

Solutions of test compounds in varying concentrations were prepared in 10 µL of dimethyl sulfoxide (DMSO) and then diluted into assay buffer (40 µL, comprising: *N,N-*bis(2-hydroxyethyl)-2-aminoethanesulfonic acid (BES), 50 mM (pH 6); polyoxyethylenesorbitan monolaurate, 0.05%; and dithiothreitol (DTT), 2.5 mM). Human cathepsin B (0.025 pMoles in 25 µL of assay buffer) was added to the dilutions. The assay solutions were mixed for 5-10 seconds on a shaker plate, covered and incubated for 30 min at room temperature. Z-FR-AMC (20 nMoles in 25 µL of assay buffer) was added to the assay solutions and hydrolysis was followed spectrophotometrically at (λ 460 nm) for 5 min. Apparent inhibition constants (Kᵢ) were calculated from the enzyme progress curves using standard mathematical models.

Compounds of the invention were tested by the above-described assay and observed to exhibit cathepsin B inhibitory activity.

### EXAMPLE 2

### Cathepsin K Assay

Solutions of test compounds in varying concentrations were prepared in 10 µL of dimethyl sulfoxide (DMSO) and then diluted into assay buffer (40 µL, comprising: MES, 50 mM (pH 5.5); EDTA, 2.5 mM; and DTT, 2.5 mM). Human cathepsin K (0.0906 pMoles in 25 µL of assay buffer) was added to the dilutions. The assay solutions were mixed for 5-10 seconds on a shaker plate, covered and incubated for 30 min at room temperature. Z-Phe-Arg-AMC (4 nMoles in 25 µL of assay buffer) was added to the assay solutions and hydrolysis was followed spectrophotometrically at (λ 460 nm) for 5 min. Apparent inhibition constants (Kᵢ) were calculated from the enzyme progress curves using standard mathematical models.

Compounds of the invention were tested by the above-described assay and observed to exhibit cathepsin K inhibitory activity.

### EXAMPLE 3

### Cathepsin L Assay

Solutions of test compounds in varying concentrations were prepared in 10 µL of dimethyl sulfoxide (DMSO) and then diluted into assay buffer (40 µL, comprising: MES, 50 mM (pH 5.5); EDTA, 2.5 mM; and DTT, 2.5 mM). Human cathepsin L (0.05 pMoles in 25 µL of assay buffer) was added to the dilutions. The assay solutions were mixed for 5-10 seconds on a shaker plate, covered and incubated for 30 min at room temperature. Z-Phe-Arg-AMC (1 nMoles in 25 µL of assay buffer) was added to the assay solutions and hydrolysis was followed spectrophotometrically at (λ 460 nm) for 5 min. Apparent inhibition constants (Kᵢ) were calculated from the enzyme progress curves using standard mathematical models.

Compounds of the invention were tested by the above-described assay and observed to exhibit cathepsin L inhibitory activity.

### EXAMPLE 4

### Cathepsin S Assay

Solutions of test compounds in varying concentrations were prepared in 10 µL of dimethyl sulfoxide (DMSO) and then diluted into assay buffer (40 µL, comprising: MES, 50 mM (pH 6.5); EDTA, 2.5 mM; and NaCl, 100 mM); β-mercaptoethanol, 2.5 mM; and BSA, 0.00%. Human cathepsin S (0.05 pMoles in 25 µL of assay buffer) was added to the dilutions. The assay solutions were mixed for 5-10 seconds on a shaker plate, covered and incubated for 30 min at room temperature. Z-Val-Val-Arg-AMC (4 nMoles in 25 µL of assay buffer containing 10% DMSO) was added to the assay solutions and hydrolysis was followed spectrophotometrically (at λ, 460 nm) for 5 min. Apparent inhibition constants (Kᵢ) were calculated from the enzyme progress curves using standard mathematical models.

Compounds of the invention were tested by the above-described assay and observed to exhibit cathepsin S inhibitory activity.

### EXAMPLE 5

### Cathepsin F Assay

Solutions of test compounds in varying concentrations were prepared in 10 µL of dimethyl sulfoxide (DMSO) and then diluted into assay buffer (40 µL, comprising: MES, 50 mM (pH 6.5); EDTA, 2.5 mM; and NaCl, 100 mM); DTT, 2.5 mM; and BSA, 0.01 %. Human cathepsin F (0.1 pMoles in 25 µL of assay buffer) was added to the dilutions. The assay solutions were mixed for 5-10 seconds on a shaker plate, covered and incubated for 30 min at room temperature. Z-Phe-Arg-AMC (2 nMoles in 25 µL of assay buffer containing 10% DMSO) was added to the assay solutions and hydrolysis was followed spectrophotometrically (at λ 460 nm) for 5 min. Apparent inhibition constants (Kᵢ) were calculated from the enzyme progress curves using standard mathematical models.

Compounds of the invention were tested by the above-described assay and observed to exhibit cathepsin F inhibitory activity.

**RESULTS**

| Compound | CATB (Ki) (M) | CATF (Ki) (M) | CATL (Ki) (M) | CATS (Ki) (M) | CATV (Ki) (M) |
|---|---|---|---|---|---|
| A | + | + | + | +++ | ++ |
| B | +++ | ++ | +++ | +++ | +++ |
| C | +++ | + | + | ++ | + |
| D | + | + | + | +++ | ++ |
| F | + | + | + | +++ | +++ |
| G | + | + | + | +++ | ++ |
| H | + | + | + | +++ | +++ |
| I | + | + | + | +++ | +++ |
| J | +++ | ++ | ++ | +++ | +++ |
| K | ++ | | +++ | +++ | +++ |
| L | + | | + | ++ | + |

| | | | | | |
|---|---|---|---|---|---|
| + indicates a Ki of more than 1 microM; ++ indicates a Ki of less than 1 microM; +++ indicates a Ki of less than 100 nM. A. (R)-N-(1-cyanocyclopropyl)-2-(2,2-difluoro-2-(4-fluorophenoxy)ethylamino)-3-(pyridin-3-ylmethylsulfonyl)propanamide; B. (S)-N-cyclopropyl-3-((R)-2-(2,2-difluoro-2-(4-fluorophenoxy)ethylamino)-3-(pyridin-3-ylmethylsulfonyl)propanamido)-2-oxopentanamide; C. (R)-N-(1-cyanocyclopropyl)-3-(cyclopropylmethylthio)-2-((S)-1,1-difluoro-1-(4-(methylthio)phenoxy)pent-3-yn-2-ylamino)propanamide; D. (R)-N-(1-cyanocyclopropyl)-3-( cyclopropylmethylsulfonyl)-2-((S)-1,1 -difluoro-1-(4-(methylsulfonyl)phenoxy)pent-3-yn-2-ylamino)propanamide; E. (R)-N-(1-cyanocyclopropyl)-2-((S)-1, 1-difluoro-1-(4-(methylthio)phenoxy)pent-3-yn-2-ylamino)-3-(pyridin-2-ylmethylthio)propanamide; F. (R)-N-(1-cyanocyclopropyl)-2-((S)-1,1-difluoro-1-(4-(methylsulfonyl)phenoxy)pent-3-yn-2-ylamino)-3-(pyridin-2-ylmethylsulfonyl)propanamide; G. (R)-N-(1-cyanocyclopropyl)-3-((S)-1,1-difluoro-1-(4-(methylthio)phenoxy)pent-3-yn-2-ylamino)-2-((pyridin-3-ylmethylthio)methyl)propanamide; H. (R)-N-(1-cyanocyclopropyl)-3-((S)-1,1 1-difluoro-1-(4-(methylsulfonyl)phenoxy)pent-3-yn-2-ylamino)-2-((pyridin-3-ylmethylsulfonyl)methyl)propanamide; I. 3-(((R)-3-(1 -cyanocyclopropylamino)-2-(((S)-1, 1-difluoro-1-(4-(methylsulfonyl)phenoxy)pent-3-yn-2-ylamino)methyl)-3-oxopropylsulfonyl)methyl)pyridine 1-oxide. J. (S)-N-cyclopropyl-3-((R)-3-(cyclopropylmethylsulfonyl)-2-(2,2-difluoro-2-(4-fluorophenoxy)ethylamino)propanamido)-2-oxopentanamide. K. (S)-5-cyclopropyl-N-((S)-1-(cyclopropylamino)-1,2-dioxopentan-3-yl)-2-(2,2-difluoro-2-(4-fluorophenoxy)ethylamino)-4,4-difluoropentanamide. L. (S)-N-(1-cyanocyclopropyl)-5-cyclopropyl-2-(2,2-difluoro-2-(4-fluorophenoxy)ethylamino)-4,4-difluoropentanamide. | | | | | |

### EXAMPLE 1

### Representative pharmaceutical formulations containing a Compound of Formula I

### ORAL FORMULATION

| | |
|---|---|
| Compound of Formula I | 10-100 mg |
| Citric Acid Monohydrate | 105 mg |
| Sodium Hydroxide | 18 mg |
| Flavoring | |
| Water | q.s. to 100 ml |

### INTRAVENOUS FORMULATION

| | |
|---|---|
| Compound of Formula I | 0.1-10 mg |
| Dextrose Monohydrate | q.s. to make isotonic |
| Citric Acid Monohydrate | 1.05 mg |
| Sodium Hydroxide | 0.18 mg |
| Water for Injection | q.s. to 1.0 ml |

### TABLET FORMULATION

| | |
|---|---|
| Compound of Formula I | 1% |
| Microcrystalline Cellulose | 73% |
| Stearic Acid | 25% |
| Colloidal Silica | 1% |

## Claims

1. A compound of Formula I: 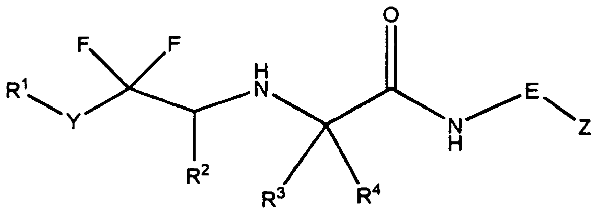 wherein:
R¹ is aryl or heteroaryl, wherein the aryl ring or heteroaryl ring is optionally substituted by one, two, or three groups independently selected from alkyl, halo, hydroxy, hydroxyalkyl, alkoxy, alkylthio, alkylsulfinyl, alkylsulfonyl, alkoxyalkyl, haloalkyl, haloalkoxy, cyano, nitro, acyl, aryl, aryloxy, arylsulfonyl, heteroaryl, heteroaryloxy, heteroarylsulfonyl, heterocyclyl, heterocyclyloxy, cycloalkyle, cycloalkyloxy, carboxy, alkoxycarbonyl, aminosulfonyl, and aminoalkyl;
Y is oxygen or -S(O)ₘ, in which m is 0, 1, or 2;
R² is hydrogen, R⁷-C≡C-, or cis or trans R⁷-CH=CH-, in which R⁷ is lower alkyl of 1-3 carbon atoms or cycloalkyl of 3-6 carbon atoms;
R³ is hydrogen or lower alkyl of 1-3 carbon atoms;
R⁴ is -A-X-R⁸, in which A is alkylene of 1-3 carbon atoms optionally substituted by one, two, or three groups independently selected from alkyl and halo, X is a covalent bond, oxygen or
-S(O)ₙ, in which n is 0, 1 or 2, and R⁸ is hydrogen, alkyl, haloalkyl, cycloalkyl, cycloalkylalkyl, aryl, aralkyl, heteroaryl, heteroaralkyl, heterocyclyl, or heterocyclylalkyl, all of which are optionally substituted with 1, 2, or 3 substituents selected from the group consisting of lower alkyl of 1-4 carbon atoms, halo, and cyano;
with the proviso that R⁸ cannot be hydrogen when X is oxygen or -S(O)ₙ;
E is a covalent bond or -CH(R⁹)-C(O)-C(O)-NH-, in which R⁹ is hydrogen or lower alkyl of 1-6 carbon atoms optionally substituted by one, two, three, or four groups independently selected from halo and cycloalkyl of 3-7 carbon atoms;
Z is -C(R⁵)(R⁶)-R¹⁰; in which
R⁵ is hydrogen or lower alkyl of 1-4 carbon atoms; and
R⁶ is hydrogen, lower alkyl of 1-4 carbon atoms, cycloalkyl, or aryl; or
R⁵ and R⁶ when taken together with the carbon atom to which they are attached form a cycloalkyl group of 3-6 carbon atoms optionally substituted by lower alkyl of 1-4 carbon atoms, halo, or hydroxyl; and;
R¹⁰ is hydrogen or cyano;
with the proviso that when E is a covalent bond R¹⁰ cannot be hydrogen; and with the proviso that when E is -CH(R⁹)-C(O)-C(O)-NH-, R¹⁰ cannot be cyano;
or a pharmaceutically acceptable salt or N-oxide thereof;
wherein each alkyl is a straight or branched, saturated aliphatic radical containing 1, 2, 3, 4, 5 or 6 carbon atoms;
each aryl is a monocyclic or fused bicyclic ring assembly containing 6, 7, 8, 9 or 10 ring carbon atoms,
each cycloalkyl is a monovalent saturated or partially unsaturated, monocyclic ring containing 3, 4, 5, 6, 7 or 8 ring carbon atoms;
each heteroaryl as a group or part of a group is an aromatic monocyclic or multicyclic moiety of 5, 6, 7, 8, 9 or 10 ring atoms in which one or more of the ring atoms is selected from nitrogen, oxygen or sulfur, the remaining atoms being carbon;
each heterocyclyl refers to a saturated or partially unsaturated, mono or bicyclic radical of 4, 5 or 6 carbon atoms wherein one or more of the ring carbon atoms are replaced by a heteroatom selected from -N=, -N-, -O-, -S-, -SO-, or -S(O)₂- and further wherein one or two ring atoms are optionally replaced by a keto (-CO-) group;
each aminoalkyl is a linear monovalent hydrocarbon radical of 1, 2, 3, 4, 5, or 6 carbon atoms or a branched monovalent hydrocarbon radical of 3, 4, 5, or 6 carbon atoms substituted with at least one -NRR' where R is hydrogen, alkyl, or- COR^{a} where R^{a} is alkyl, and R' is hydrogen or alkyl;
each aminosulfonyl is an -SO₂R radical where R is -NRR' where R is hydrogen, alkyl, or -COR^{a} where R^{a} is alkyl, and R' hydrogen or alkyl as defined above;
each aminocarbonyl is a -CONRR' radical where R is hydrogen or alkyl and R' hydrogen, alkyl, aryl, aralkyl, heteroaryl, or heteroaralkyl.

2. The compound of claim 1, wherein R¹ is optionally substituted aryl and Y is oxygen.

3. The compound of claim 2, wherein either:
(a) R² and R³ are both hydrogen and E is a covalent bond; or
(b) E is -CH(R⁹)-C(O)-C(O)-NH-; or
(c) R² is R⁷-C≡C-, R³ is hydrogen, and E is a covalent bond.

4. The compound of claim 3, wherein Z is -C(R⁵)(R⁶)-R¹⁰, in which R⁵ and R⁶ together with the carbon atom to which they are attached form a cycloalkyl group of 3-6 carbon atoms.

5. The compound of claim 4, wherein R² and R³ are both hydrogen, E is a covalent bond, the cycloalkyl group is cyclopropyl, and R¹⁰ is cyano.

6. The compound of claim 5, wherein R¹ is phenyl substituted by fluoro, A is alkylene of 1-3 carbon atoms optionally substituted by fluoro, X is a covalent bond or - S(O)₂, and R⁸ is heteroarylalkyl or cycloalkyalkyl.

7. The compound of claim 6, wherein the compound is selected from the group consisting of (S)-N-(1-cyanocyclopropyl)-5-cyclopropyl-2-(2,2-difluoro-2-(4-fluorophenoxy)ethylamino)-4,4-difluoropentanamide and (R)-N-(1-cyanocyclopropyl)-2-(2,2-difluoro-2-(4-fluorophenoxy)ethylamino)-3-(pyridin-3-ylmethylsulfonyl)propanamide.

8. The compound of claim 2, wherein the cycloalkyl group is cyclopropyl, R¹⁰ is hydrogen, and either:
(a) E is -CH(R⁹-C(O)-C(O)-NH-, Z is -C(R⁵)(R⁶)-R¹⁰, in which R⁵ and R⁶ together with the carbon atom to which they are attached form a cycloalkyl group of 3-6 carbon atoms; or
(b) R² is R⁷-C≡C-, R³ is hydrogen and E is a covalent bond, and optionally wherein R⁷ is methyl.

9. The compound of claim 8, wherein either:
(a) R¹ is phenyl substituted by fluoro, A is alkylene of 1-3 carbon atoms optionally substituted by fluoro, X is a covalent bond or -S(O)₂, R⁸ is heteroaryl or cycloalkyl, and R⁹ is ethyl, when E is -CH(R⁹-C(O)-C(O)-NH-; or
(b) R¹ is phenyl substituted by fluoro, thiomethyl, or methylsulfonyl, A is alkylene of 1-3 carbon atoms optionally substituted by fluoro, X is a covalent bond or ―S(O)₂, and R⁸ is heteroarylalkyl or cycloalkyl, when R² is R⁷-C≡C-, R³ is hydrogen and E is a covalent bond.

10. The compound of claim 9, wherein the compound is selected from the group consisting of (S)-5-cyclopropyl-N-((S)-1-(cyclopropylamino)-1,2-dioxopentan-3-yl)-2-(2,2-difluoro-2-(4-fluorophenoxy)ethylamino)-4,4-difluoropentanamide, (S)-N-cyclopropyl-3-((R)-2-(2,2-difluoro-2-(4-fluorophenoxy)ethylamino)-3-(pyridin-3-ylmethylsulfonyl)propanamido)-2-oxopentanamide, and (S)-N-cyclopropyl-3-((R)-3-(cyclopropylmethylsulfonyl)-2-(2,2-difluoro-2-(4-fluorophenoxy)-ethylamino)propanamido)-2-oxopentanamide.

11. The compound of claim 9, selected from the group consisting of (R)-N-( 1-cyanocyclopropyl)-3-(cyclopropylmethylthio)-2-((S)-1,1-difluoro-1-(4-(methylthio)phenoxy)pent-3-yn-2-ylamino)propanamide, (R)-N-(1-cyanocyclopropyl)-3-(cyclopropylmethylsulfonyl)-2-((S)-1,1-difluoro-1-(4-(methylsulfonyl)phenoxy)pent-3-yn-2-ylamino)propanamide, (R)-N-(1-cyanocyclopropyl)-2-((S)-1,1-difluoro-1-(4-(methylthio)phenoxy)pent-3-yn-2-ylamino)-3-(pyridin-2-ylmethylthio)propanamide, (R)-N-(1-cyanocyclopropyl)-2-((S)-1,1-difluoro-1-(4-(methylsulfonyl)phenoxy)pent-3-yn-2-ylamino)-3-(pyridin-2-ylmethylsulfonyl)propanamide, (R)-N-(1-cyanocyclopropyl)-3-((S)-1,1-difluoro-1-(4-(methylthio)phenoxy)pent-3-yn-2-ylamino)-2-((pyridin-3-ylmethylthio)methyl)propanamide, (R)-N-(1-cyanocyclopropyl)-3-((S)-1,1-difluoro-1-(4-(methylsulfonyl)phenoxy)pent-3-yn-2-ylamino)-2-((pyridin-3-ylmethylsulfonyl)methyl)propanamide, and 3-(((R)-3-(1-cyanocyclopropylamino)-2-(((S)-1,1-difluoro-1-(4-(methylsulfonyl)phenoxy)pent-3-yn-2-ylamino)methyl)-3-oxopropylsulfonyl)methyl)pyridine 1-oxide.

12. A compound according to any one of claims 1 to 11 for use in the treatment of the human or animal body by therapy.

13. A pharmaceutical composition comprising a compound according to any one of claims 1 to 11 in admixture with one or more suitable excipients.

14. A pharmaceutical composition comprising a compound according to any one of claims 1 to 11 or a pharmaceutically acceptable salt thereof for use in a method for treating a disease in an animal mediated by Cathepsin B, K, L, F, or S, which method comprises administering to the animal said pharmaceutical composition optionally in admixture with one or more suitable excipients.

15. The pharmaceutical composition of claim 14 wherein the disease is asthma, arthritis, atherosclerosis, COPD, MS, or psoriasis.

## Patentansprüche

1. Verbindung der Formel I: 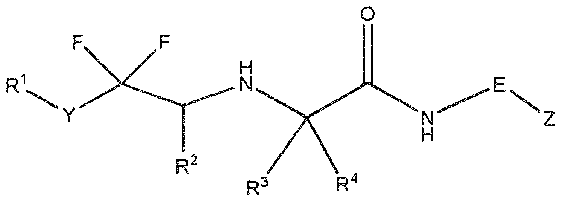 worin:
R¹ Aryl oder Heteroaryl ist, wobei der Arylring oder Heteroarylring gegebenenfalls mit einer, zwei oder drei Gruppen substituiert ist, die unabhängig ausgewählt sind aus Alkyl, Halogen, Hydroxy, Hydroxyalkyl, Alkoxy, Alkylthio, Alkylsulfinyl, Alkylsulfonyl, Alkoxyalkyl, Halogenalkyl, Halogenalkoxy, Cyano, Nitro, Acyl, Aryl, Aryloxy, Arylsulfonyl, Heteroaryl, Heteroaryloxy, Heteroarylsulfonyl, Heterocyclyl, Heterocyclyloxy, Cycloalkyl, Cycloalkyloxy, Carboxy, Alkoxycarbonyl, Aminosulfonyl und Aminoalkyl;
Y Sauerstoff oder -S(O)m, worin m 0, 1 oder 2 ist, ist;
R² Wasserstoff, R⁷-C≡C- oder cis- oder trans-R⁷-CH=CH-ist, worin R⁷ Niederalkyl mit 1-3 Kohlenstoffatomen oder Cycloalkyl mit 3-6 Kohlenstoffatomen ist;
R³ Wasserstoff oder Niederalkyl mit 1-3 Kohlenstoffatomen ist;
R⁴ -A-X-R⁸ ist, worin A Alkylen mit 1-3 Kohlenstoffatomen, das gegebenenfalls mit einer, zwei oder drei Gruppen, unabhängig ausgewählt aus Alkyl und Halogen, substituiert ist, ist, X eine kovalente Bindung, Sauerstoff oder -S(O)ₙ, worin n 0, 1 oder 2 ist, ist und R" Wasserstoff, Alkyl, Halogenalkyl, Cycloalkyl, Cycloalkylalkyl, Aryl, Aralkyl, Heteroaryl, Heteroaralkyl, Heterocyclyl oder Heterocyclylalkyl ist, die alle gegebenenfalls mit 1, 2 oder 3 Substituenten, ausgewählt aus der Gruppe, bestehend aus Niederalkyl mit 1-4 Kohlenstoffatomen, Halogen und Cyano, substituiert sind;
mit der Maßgabe, dass R" nicht Wasserstoff sein kann, wenn X Sauerstoff oder -S(O)ₙ ist;
E eine kovalente Bindung oder -CH(R⁹)-C(O)-C(O)-NH- ist, wobei R⁹ Wasserstoff oder Niederalkyl mit 1-6 Kohlenstoffatomen ist, das gegebenenfalls mit einer, zwei, drei oder vier Gruppen, unabhängig aus Halogen und Cycloalkyl mit 3-7 Kohlenstoffatomen ausgewählt, substituiert ist;
Z -C(R⁵) (R⁶)-R¹⁰ ist, worin
R⁵ Wasserstoff oder Niederalkyl mit 1-4 Kohlenstoffatomen ist und
R⁶ Wasserstoff, Niederalkyl mit 1-4 Kohlenstoffatomen, Cycloalkyl oder Aryl ist oder
R⁵ und R⁶, wenn sie mit dem Kohlenstoffatom, an das sie gebunden sind, zusammengenommen werden, eine Cycloalkylgruppe mit 3-6 Kohlenstoffatomen bilden, die gegebenenfalls mit Niederalkyl mit 1-4 Kohlenstoffatomen, Halogen oder Hydroxyl substituiert ist, und
R¹⁰ Wasserstoff oder Cyano ist,
mit der Maßgabe, dass, wenn E eine kovalente Bindung ist, R¹⁰ nicht Wasserstoff sein kann, und mit der Maßgabe, dass, wenn E -CH(R⁹)-C(O)-C(O)-NH- ist, R¹⁰ nicht Cyano sein kann,
oder ein pharmazeutisches annehmbares Salz oder N-Oxid davon;
wobei jedes Alkyl ein geradkettiger oder verzweigter, gesättigter aliphatischer Rest ist, der 1, 2, 3, 4, 5 oder 6 Kohlenstoffatome enthält;
jedes Aryl eine monocyclische oder kondensierte bicyclische Ringanordnung ist, die 6, 7, 8, 9 oder 10 Ringatome enthält,
jedes Cycloalkyl ein monovalenter gesättigter oder partiell ungesättigter monocyclischer Ring ist, der 3, 4, 5, 6, 7 oder 8 Ringatome enthält;
jedes Heteroaryl als Gruppe oder Teil einer Gruppe eine aromatische monocyclische oder multicyclische Gruppierung mit 5, 6, 7, 8, 9 oder 10 Ringatomen ist, in der ein oder mehrere der Ringatome aus Stickstoff, Sauerstoff oder Schwefel ausgewählt ist/sind, wobei die restlichen Atome Kohlenstoff sind;
jedes Heterocyclyl sich auf einen gesättigten oder partiell ungesättigten, mono- oder bicyclischen Rest mit 4, 5, oder 6 Kohlenstoffatomen bezieht, wobei eins oder mehrere der Ringatome durch ein Heteroatom, ausgewählt aus -N=, -N-, -O-, -S-, -SO- oder -S(O)₂-, ersetzt ist/sind und worin außerdem ein oder zwei Ringatom(e) gegebenenfalls durch eine Keto (-CO-)-Gruppe ersetzt ist/sind;
jedes Aminoalkyl ein linearer monovalenter Kohlenwasserstoffrest mit 1, 2, 3, 4, 5 oder 6 Kohlenstoffatomen oder ein verzweigter monovalenter Kohlenwasserstoffrest mit 3, 4, 5 oder 6 Kohlenstoffatomen ist, der mit wenigstens einem -NRR*'* substituiert ist, worin R Wasserstoff, Alkyl oder -COR^{a} ist, worin R^{a} Alkyl ist und R*'* Wasserstoff oder Alkyl ist;
jedes Aminosulfonyl ein -SO₂R-Rest ist, worin R -NRR*'* ist, worin R Wasserstoff, Alkyl oder -COR^{a} ist, worin R^{a} Alkyl ist und R*'* Wasserstoff oder Alkyl, wie oben definiert, ist;
jedes Aminocarbonyl ein -CONRR*'*-Rest ist, worin R Wasserstoff oder Alkyl ist und R*'* Wasserstoff, Alkyl, Aryl, Aralkyl, Heteroaryl oder Heteroaralkyl ist.

2. Verbindung gemäß Anspruch 1, wobei R¹ gegebenenfalls substituiertes Aryl ist und Y Sauerstoff ist.

3. Verbindung gemäß Anspruch 2, wobei entweder:
(a) R² und R³ beide Wasserstoff sind und E eine kovalente Bindung ist oder
(b) E -CH(R⁹)-C(O)-C(O)-NH- ist oder
(c) R² R⁷-C≡C- ist, R³ Wasserstoff ist und E eine kovalente Bindung ist.

4. Verbindung gemäß Anspruch 3, wobei Z -C(R⁵) (R⁶)-R¹⁰ ist, worin R⁵ und R⁶ zusammen mit dem Kohlenstoffatom, an das sie gebunden sind, eine Cycloalkylgruppe mit 3-6 Kohlenstoffatomen bilden.

5. Verbindung gemäß Anspruch 4, wobei R² und R³ beide Wasserstoff sind, E eine kovalente Bindung ist, die Cycloalkylgruppe Cyclopropyl ist und R¹⁰ Cyano ist.

6. Verbindung gemäß Anspruch 5, wobei R¹ mit Fluor substituiertes Phenyl ist, A Alkylen mit 1-3 Kohlenstoffatomen ist, das gegebenenfalls mit Fluor substituiert ist, X eine kovalente Bindung oder -S(O)₂ ist und R⁸ Heteroarylalkyl oder Cycloalkylalkyl ist.

7. Verbindung gemäß Anspruch 6, wobei die Verbindung aus der Gruppe, bestehend aus (S)-N-(1-Cyanocyclopropyl)-5-cyclopropyl-2-(2,2-difluor-2-(4-fluorphenoxy)ethylamino)-4,4-difluor-pentanamid und (R)-N-(1-Cyanocyclopropyl)-2-(2,2-difluor-2-(4-fluorphenoxy)ethylamino)-3-(pyridin-3-ylmethylsulfonyl)-propanamid, ausgewählt ist.

8. Verbindung gemäß Anspruch 2, wobei die Cycloalkylgruppe Cyclopropyl ist, R¹⁰ Wasserstoff ist und entweder:
(a) E -CH(R⁹)-C(O)-C(O)-NH- ist, Z -C(R⁵) (R⁶)-R¹⁰ ist, worin R⁵ und R⁶ zusammen mit dem Kohlenstoffatom, an das sie gebunden sind, eine Cycloalkylgruppe mit 3-6 Kohlenstoffatomen bilden, oder
(b) R² R⁷-C≡C- ist, R³ Wasserstoff ist und E eine kovalente Bindung ist und wobei gegebenenfalls R⁷ Methyl ist.

9. Verbindung gemäß Anspruch 8, wobei entweder:
(a) R¹ mit Fluor substituiertes Phenyl ist, A Alkylen mit 1-3 Kohlenstoffatomen, das gegebenenfalls mit Fluor substituiert ist, ist, X eine kovalente Bindung oder -S(O)₂ ist, R⁸ Heteroaryl oder Cycloalkyl ist und R⁹ Ethyl ist, wenn E -CH(R⁹)-C(O)-C(O)-NH- ist; oder
(b) R¹ mit Fluor substituiertes Phenyl, Thiomethyl oder Methylsulfonyl ist, A Alkylen mit 1-3 Kohlenstoffatomen, das gegebenenfalls mit Fluor substituiert ist, ist, X eine kovalente Bindung oder -S(O)₂ ist und R⁸ Heteroarylalkyl oder Cycloalkyl ist, wenn R² R⁷-C≡C- ist, R³ Wasserstoff ist und E eine kovalente Bindung ist.

10. Verbindung gemäß Anspruch 9, wobei die Verbindung aus der Gruppe, bestehend aus (S)-5-Cyclopropyl-N-((S)-1-(cyclopro-pylamino)-1,2-dioxopentan-3-yl)-2-(2,2-difluor-2-(4-fluor-phenoxy)ethylamino)-4,4-difluorpentanamid, (S)-N-Cyclopropyl-3-((R)-2-(2,2-difluor-2-(4-fluorphenoxy)ethylamino)-3-(pyridin-3-ylmethylsulfonyl)propanamido)-2-oxopentanamid und (S)-N-Cyclopropyl-3-((R)-3-(cyclopropylmethylsulfonyl)-2-(2,2-difluor-2-(4-fluorphenoxy)ethylamino)propanamido)-2-oxopentanamid, ausgewählt ist.

11. Verbindung gemäß Anspruch 9, die aus der Gruppe, bestehend aus (R)-N-(1-Cyanocyclopropyl)-3-(cyclopropylmethylthio)-2-((S)-1,1-difluor-1-(4-(methylthio)phenoxy)pent-3-in-2-ylamino)propanamid, (R)-N-(1-Cyanocyclopropyl)-3-(cyclopropylmethylsulfonyl)-2-((S)-1,1-difluor-1-(4-(methylsulfonyl)phenoxy)pent-3-in-2-ylamino)propanamid, (R)-N-(1-Cyanocyclopropyl)-2-((S)-1,1-difluor-1-(4-(methylthio)phenoxy)pent-3-in-2-ylamino)-3-(pyridin-2-ylmethylthio)propanamid, (R)-N-(1-Cyanocyclopropyl)-2-((S)-1,1-difluor-1-(4-(methylsulfonyl)-phenoxy)pent-3-in-2-ylamino)-3-(pyridin-2-ylmethylsulfonyl)-propanamid, (R)-N-(1-Cyanocyclopropyl)-3-((S)-1,1-difluor-1-(4-(methylthio)phenoxy)pent-3-in-2-ylamino)-2-((pyridin-3-ylmethylthio)methyl)propanamid, (R)-N-(1-Cyanocyclopropyl)-3-((S)-1,1-difluor-1-(4-(methylsulfonyl)phenoxy)pent-3-in-2-ylamino)-2-((pyridin-3-ylmethylsulfonyl)methyl)propanamid und 3-(((R)-3-(1-Cyanocyclopropylamino)-2-(((S)-1,1-difluor-1-(4-(methylsulfonyl)phenoxy)pent-3-in-2-ylamino)methyl)-3-oxopropylsulfonyl)methyl)pyridin-1-oxid, ausgewählt ist.

12. Verbindung gemäß einem der Ansprüche 1 bis 11 zur Verwendung bei der Behandlung des Menschen- oder Tierkörpers durch Therapie.

13. Pharmazeutische Zusammensetzung, die eine Verbindung gemäß einem der Ansprüche 1 bis 11 im Gemisch mit einem geeigneten Exzipiens oder mehreren geeigneten Exzipientien umfasst.

14. Pharmazeutische Zusammensetzung, die eine Verbindung gemäß einem der Ansprüche 1 bis 11 oder ein pharmazeutisch annehmbares Salz davon umfasst, zur Verwendung in einem Verfahren zur Behandlung einer Krankheit bei einem Tier, die durch Cathepsin B, K, L, F oder S vermittelt wird, wobei das Verfahren Verabreichen der pharmazeutischen Zusammensetzung, gegebenenfalls im Gemisch mit einem geeigneten Exzipiens oder mehreren geeigneten Exzipientien, an das Tier umfasst.

15. Pharmazeutische Zusammensetzung gemäß Anspruch 14, wobei die Krankheit Asthma, Arthritis, Atherosklerose, COPD, MS oder Psoriasis ist.

## Revendications

1. Composé de formule I : 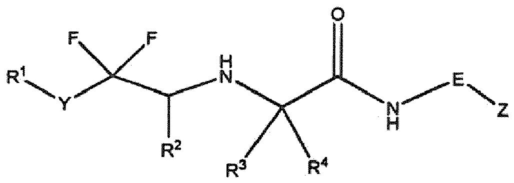 dans laquelle
- R¹ représente un groupe aryle ou hétéroaryle, dont le cycle aryle ou hétéroaryle peut, en option, porter un, deux ou trois substituant(s) choisi(s) indépendamment parmi les atomes d'halogène et les groupes alkyle, hydroxyle, hydroxy-alkyle, alcoxy, alkylthio, alkyl-sulfinyle, alkyl-sulfonyle, alcoxy-alkyle, halogéno-alkyle, halogéno-alcoxy, cyano, nitro, acyle, aryle, aryl-oxy, aryl-sulfonyle, hétéroaryle, hétéroaryl-oxy, hétéroaryl-sulfonyle, hétérocyclyle, hétérocyclyl-oxy, cycloalkyle, cycloalkyl-oxy, carboxyle, alcoxy-carbonyle, amino-sulfonyle et amino-alkyle ;
- Y représente un chaînon constitué par un atome d'oxygène ou symbolisé par -S(O)ₘ- où l'indice m vaut 0, 1 ou 2 ;
- R² représente un atome d'hydrogène ou un groupe symbolisé par R⁷-C≡C-, ou R⁷-C=C- en configuration cis ou trans, où R⁷ représente un groupe alkyle inférieur comportant de 1 à 3 atomes de carbone ou un groupe cycloalkyle comportant de 3 à 6 atomes de carbone ;
- R³ représente un atome d'hydrogène ou un groupe alkyle inférieur comportant de 1 à 3 atomes de carbone ;
- R⁴ représente un groupe symbolisé par -A-X-R⁸, où
- A représente un groupe alcanediyle comportant de 1 à 3 atomes de carbone et pouvant, en option, porter un, deux ou trois substituant(s) choisi(s) indépendamment parmi les groupes alkyle et les atomes d'halogène,
- X représente une liaison covalente ou un chaînon constitué par un atome d'oxygène ou symbolisé par -S(O)ₙ- où l'indice n vaut 0, 1 ou 2,
- et R⁸ représente un atome d'hydrogène ou un groupe alkyle, halogéno-alkyle, cycloalkyle, cycloalkyl-alkyle, aryle, aryl-alkyle, hétéroaryle, hétéroaryl-alkyle, hétérocyclyle ou hétérocyclyl-alkyle, lesquels groupes peuvent tous, en option, porter un, deux ou trois substituant(s) choisi(s) parmi les groupes alkyle inférieur comportant de 1 à 4 atomes de carbone, les atomes d'halogène et le groupe cyano,
sous réserve que, si X représente un chaînon constitué par un atome d'oxygène ou symbolisé par -S(O)ₙ-, R⁸ ne représente pas un atome d'hydrogène ;
- E représente une liaison covalente ou un raccord symbolisé par -CH(R⁹)-C(O)-C(O)-NH-, où R⁹ représente un atome d'hydrogène ou un groupe alkyle inférieur comportant de 1 à 6 atomes de carbone et pouvant, en option, porter un, deux, trois ou quatre substituant(s) choisi(s) indépendamment parmi les atomes d'halogène et les groupes cycloalkyle comportant de 3 à 7 atomes de carbone ;
- et Z représente un groupe de formule -C(R⁵)(R⁶)-R¹⁰ dans laquelle
- R⁵ représente un atome d'hydrogène ou un groupe alkyle inférieur comportant de 1 à 4 atomes de carbone,
- R⁶ représente un atome d'hydrogène ou un groupe alkyle inférieur comportant de 1 à 4 atomes de carbone, cycloalkyle ou aryle,
- ou bien R⁵ et R⁶ représentent des entités qui, conjointement avec l'atome de carbone auquel elles sont liées, forment un groupe cycloalkyle comportant de 3 à 6 atomes de carbone et pouvant, en option, porter un ou des substituant(s) alkyle inférieur comportant de 1 à 4 atomes de carbone, halogéno ou hydroxy ;
- et R¹⁰ représente un atome d'hydrogène ou un groupe cyano, sous réserve que, si E représente une liaison covalente, R¹⁰ ne représente pas un atome d'hydrogène, et que, si E représente un raccord symbolisé par -CH(R⁹)-C(O)-C(O)-NH-, R¹⁰ ne représente pas un groupe cyano ;
ou sel ou N-oxyde pharmacologiquement admissible d'un tel composé, étant entendu que :
- tout groupe alkyle est un groupe aliphatique saturé, à chaîne droite ou ramifiée, comportant 1, 2, 3, 4, 5 ou 6 atome(s) de carbone,
- tout groupe aryle est un groupe monocyclique ou bicyclique à cycles condensés, comportant 6, 7, 8, 9 ou 10 atomes de carbone en tant que chaînons de cycle,
- tout groupe cycloalkyle est un groupe monocyclique monovalent, saturé ou partiellement insaturé, comportant 3, 4, 5, 6, 7 ou 8 atomes de carbone en tant que chaînons de cycle,
- tout groupe hétéroaryle, constituant un groupe à lui seul ou une partie d'un groupe, est un groupe monocyclique ou polycyclique aromatique, comportant 5, 6, 7, 8, 9 ou 10 atomes en tant que chaînons de cycle, dans lequel l'un ou plusieurs de ces atomes chaînons de cycle est ou sont choisi(s) parmi les atomes d'azote, d'oxygène et de soufre, le reste des atomes étant un ou des atomes de carbone,
- tout groupe hétérocyclyle est un groupe monocyclique ou bicyclique, saturé ou partiellement insaturé, comportant 4, 5 ou 6 atomes de carbone, dans lequel l'un ou plusieurs de ces atomes de carbone chaînons de cycle est ou sont chacun remplacé(s) par un chaînon hétéroatomique choisi parmi ceux qui sont symbolisés par -N=, -N-, -O-, -S-, -SO- et -S(O)₂-, et dans lequel, en outre, un ou deux atomes chaînons de cycle peut ou peuvent être chacun, en option, remplacé(s) par un groupe oxo -C(O)-,
- tout groupe amino-alkyle est un groupe hydrocarbyle monovalent à chaîne linéaire comportant 1, 2, 3, 4, 5 ou 6 atome(s) de carbone, ou un groupe hydrocarbyle monovalent à chaîne ramifiée comportant 3, 4, 5 ou 6 atomes de carbone, qui porte au moins un substituant symbolisé par -NRR' où R représente un atome d'hydrogène, un groupe alkyle ou un groupe symbolisé par -COR^{a} où R^{a} représente un groupe alkyle, et R' représente un atome d'hydrogène ou un groupe alkyle,
- tout groupe amino-sulfonyle est un groupe symbolisé par -SO₂R où R représente un groupe symbolisé par -NRR' où R représente un atome d'hydrogène, un groupe alkyle ou un groupe symbolisé par -COR^{a} où R^{a} représente un groupe alkyle, et R' représente un atome d'hydrogène ou un groupe alkyle tel que défini plus haut,
- et tout groupe amino-carbonyle est un groupe de formule -CONRR' dans laquelle R représente un atome d'hydrogène ou un groupe alkyle et R' représente un atome d'hydrogène ou un groupe alkyle, aryle, aryl-alkyle, hétéroaryle ou hétéroaryl-alkyle.

2. Composé conforme à la revendication 1, dans lequel R¹ représente un groupe aryle, en option porteur d'un ou de substituant(s), et Y représente un atome d'oxygène.

3. Composé conforme à la revendication 2, dans lequel
a) soit R² et R³ représentent tous les deux des atomes d'hydrogène et E représente une liaison covalente,
b) soit E représente un raccord -CH(R⁹)-C(O)-C(O)-NH-,
c) soit R² représente un groupe R⁷-C≡C-, R³ représente un atome d'hydrogène et E représente une liaison covalente.

4. Composé conforme à la revendication 3, dans lequel Z représente un groupe de formule -C(R⁵)(R⁶)-R¹⁰ dans laquelle R⁵ et R⁶ représentent des entités qui, conjointement avec l'atome de carbone auquel elles sont liées, forment un groupe cycloalkyle comportant de 3 à 6 atomes de carbone.

5. Composé conforme à la revendication 4, dans lequel R² et R³ représentent tous les deux des atomes d'hydrogène, E représente une liaison covalente, le groupe cycloalkyle est un groupe cyclopropyle, et R¹⁰ représente un groupe cyano.

6. Composé conforme à la revendication 5, dans lequel R¹ représente un groupe phényle porteur d'un ou de substituant(s) fluoro, A représente un groupe alcanediyle comportant de 1 à 3 atomes de carbone, en option, porteur d'un ou de substituant(s) fluoro, X représente une liaison covalente ou un chaînon -S(O)₂-, et R⁸ représente un groupe hétéroaryl-alkyle ou cycloalkyl-alkyle.

7. Composé conforme à la revendication 6, lequel composé est choisi dans l'ensemble formé par les suivants :
- (S)-N-(1-cyano-cyclopropyl)-5-cyclopropyl-2-[2,2-difluoro-2-(4-fluoro-phénoxy)-éthyl-amino]-4,4-difluoro-pentanamide,
- (R)-N-(1-cyano-cyclopropyl)-2-[2,2-difluoro-2-(4-fluoro-phénoxy)-éthyl-amino]-3-[(pyridin-3-yl)-méthyl-sulfonyl]-propanamide.

8. Composé conforme à la revendication 2, dans lequel le groupe cycloalkyle est un groupe cyclopropyle, R¹⁰ représente un atome d'hydrogène, et
a) soit E représente un raccord -CH(R⁹)-C(O)-C(O)-NH-, et Z représente un groupe de formule -C(R⁵)(R⁶)-R¹⁰ dans laquelle R⁵ et R⁶ représentent des entités qui, conjointement avec l'atome de carbone auquel elles sont liées, forment un groupe cycloalkyle comportant de 3 à 6 atomes de carbone,
b) soit R² représente un groupe R⁷-C≡C-, R³ représente un atome d'hydrogène et E représente une liaison covalente, et en option, R⁷ représente un groupe méthyle.

9. Composé conforme à la revendication 8, dans lequel
a) soit R¹ représente un groupe phényle porteur d'un ou de substituant(s) fluoro, A représente un groupe alcanediyle comportant de 1 à 3 atomes de carbone et pouvant, en option, porter un ou des substituant(s) fluoro, X représente une liaison covalente ou un chaînon -S(O)₂-, R⁸ représente un groupe hétéroaryle ou cycloalkyle, et R⁹ représente un groupe éthyle, si E représente un fragment de formule -CH(R⁹)-C(O)-C(O)-NH-,
b) soit R¹ représente un groupe phényle porteur d'un ou de substituant(s) fluoro, méthyl-thio ou méthyl-sulfonyle, A représente un groupe alcanediyle comportant de 1 à 3 atomes de carbone et pouvant, en option, porter un ou des substituant(s) fluoro, X représente une liaison covalente ou un chaînon -S(O)₂-, et R⁸ représente un groupe hétéroaryl-alkyle ou cycloalkyle, si R² représente un groupe R⁷-C≡C-, R³ représente un atome d'hydrogène et E représente une liaison covalente.

10. Composé conforme à la revendication 9, lequel composé est choisi dans le groupe formé par les suivants :
- (S)-5-cyclopropyl-N-[(S)-1-(cyclopropyl-amino)-1,2-dioxo-pentan-3-yl]-2-[2,2-difluoro-2-(4-fluoro-phénoxy)-éthyl-amino]-4,4-di-fluoro-pentanamide,
- (S)-N-cyclopropyl-3-{(R)-2-[2,2-difluoro-2-(4-fluoro-phénoxy)-éthyl-amino]-3-[(pyridin-3-yl)-méthyl-sulfonyl]-propanamido}-2-oxo-pentanamide,
- (S)-N-cyclopropyl-3-{(R)-2-[2,2-difluoro-2-(4-fluoro-phénoxy)-éthyl-amino]-3-(cyclopropyl-méthyl-sulfonyl)-propanamido}-2-oxo-pentanamide.

11. Composé conforme à la revendication 9, lequel composé est choisi dans le groupe formé par les suivants :
- (R)-N-(1-cyano-cyclopropyl)-3-(cyclopropyl-méthylthio)-2-[(S)-1,1-difluoro-1-(4-méthylthio-phénoxy)-pent-3-yn-2-yl-amino]-propanamide,
- (R)-N-(1-cyano-cyclopropyl)-3-(cyclopropyl-méthyl-sulfonyl)-2-{(S)-1,1-difluoro-1-[(4-(méthyl-sulfonyl)-phénoxy]-pent-3-yn-2-yl-amino}-propanamide,
- (R)-N-(1-cyano-cyclopropyl)-2-[(S)-1,1-difluoro-1-(4-méthylthio-phénoxy)-pent-3-yn-2-yl-amino]-3-[(pyridin-2-yl)-méthylthio]-propanamide,
- (R)-N-(1-cyano-cyclopropyl)-2-{(S)-1,1-difluoro-1-[(4-(méthyl-sulfonyl)-phénoxy]-pent-3-yn-2-yl-amino}-3-[(pyridin-2-yl)-méthyl-sulfonyl]-propanamide,
- (R)-N-(1-cyano-cyclopropyl)-3-[(S)-1,1-difluoro-1-(4-méthylthio-phénoxy)-pent-3-yn-2-yl-amino]-2-{[(pyridin-3-yl)-méthylthio]-méthyl}-propanamide,
- (R)-N-(1-cyano-cyclopropyl)-3-{(S)-1,1-difluoro-1-[(4-(méthyl-sulfonyl)-phénoxy]-pent-3-yn-2-yl-amino}-2-{[(pyridin-3-yl)-méthyl-sulfonyl]-méthyl}-propanamide,
- 3-({[(R)-3-[(1-cyano-cyclopropyl)-amino]-2-({(S)-1,1-difluoro-1-[4-(méthyl-sulfonyl)-phénoxy]-pent-3-yn-2-yl-amino}-méthyl)-3-oxo-propyl]-sulfonyl -méthyl)-pyridine-1-oxyde.

12. Composé conforme à l'une des revendications 1 à 11 pour utilisation dans un traitement thérapeutique du corps d'un humain ou d'un animal.

13. Composition pharmaceutique comprenant un composé conforme à l'une des revendications 1 à 11, mélangé avec un ou plusieurs excipient(s) approprié(s).

14. Composition pharmaceutique comprenant un composé conforme à l'une des revendications 1 à 11 ou un sel pharmacologiquement admissible d'un tel composé, en option mélangé avec un ou plusieurs excipient(s) approprié(s), pour utilisation dans un procédé de traitement, chez un animal, d'une maladie où intervient une cathepsine B, K, L, F ou S, lequel procédé comporte le fait d'administrer à l'animal ladite composition pharmaceutique.

15. Composition pharmaceutique conforme à la revendication 14, pour laquelle la maladie est un asthme, une arthrite, une athérosclérose, une bronchopneumopathie chronique obstructive, une sclérose en plaques, ou un psoriasis.
